(19) Europäisches Patentamt
European Patent Office
Office européen des brevets

(11) EP 0 685 465 B1

(12) **EUROPÄISCHE PATENTSCHRIFT**

(45) Veröffentlichungstag und Bekanntmachung des Hinweises auf die Patenterteilung:
**10.01.2001 Patentblatt 2001/02**

(51) Int Cl.⁷: **C07D 211/46**, C08K 5/3435, C07D 211/58, C07D 491/10, C07D 471/10

(21) Anmeldenummer: **95810345.9**

(22) Anmeldetag: **23.05.1995**

# (54) Butin- oder Hexadiin-verknüpfte gehinderte Amine als Stabilisatoren

Butine- or hexadiine linked hindered amines as stabilizer

Amines encomprées liées par un groupe butine ou hexadiine comme stabilisateurs

(84) Benannte Vertragsstaaten:
**DE FR GB IT**

(30) Priorität: **02.06.1994 CH 173394**

(43) Veröffentlichungstag der Anmeldung:
**06.12.1995 Patentblatt 1995/49**

(73) Patentinhaber: **Ciba Specialty Chemicals Holding Inc.**
**4057 Basel (CH)**

(72) Erfinder: **Steinmann, Alfred, Dr.**
**CH-1724 Praroman (CH)**

(56) Entgegenhaltungen:
**US-A- 3 085 093** **US-A- 3 755 586**
**US-A- 3 940 363** **US-A- 4 386 127**

- **J. ORG. CHEM., Bd.27, Nr.4, 12. April 1962 Seiten 1695 - 1703 W. B. LUTZ ET. AL. 'New Derivatives of 2,2,6,6-Tetramethylpiperidine'**

Bemerkungen:
Die Akte enthält technische Angaben, die nach dem Eingang der Anmeldung eingereicht wurden und die nicht in dieser Patentschrift enthalten sind.

EP 0 685 465 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Gegenstand der Erfindung sind neue Verbindungen, in denen 2 sterisch gehinderte Amineinheiten über die Stickstoffatome endständig mit einer 2-Butin oder 2,4-Hexadiineinheit verbunden sind, deren Verwendung zum Stabilisieren von organischem Material gegen oxidativen, thermischen oder aktinischen Abbau, entsprechende Zusammensetzungen und ein Verfahren zum Stabilisieren, sowie ein neues Herstellungsverfahren für die neuen Verbindungen.

Eine Reihe von gehinderten Aminen vom Typ des 2,2,6,6-Tetramethylpiperidins sind bereits als Zusatzstoff für synthetische Polymere, besonders als Lichtschutzmittel, bekannt; darunter auch in 4-Stellung substituierte Derivate von 1-Propargyl-2,2,6,6-tetramethylpiperidin. Beispielhaft seien dazu die folgenden Publikationen genannt: US-A-4472547; EP-A-11051 (C.A.94:39518r); DE-A-2805838 (C.A.90:22820c). US-A-4386127 beschreibt auch 1-Butinyl-2,2,6,6-tetramethyl-4-hydroxypiperidin.

Auch die Verwendung einiger Verbindungen vom Typ 2,6-Diarylpiperidin-1-yl-substituiertes 2-Buten als Stabilisatoren ist bekannt (US-A-5204474).

Eine Reihe von wissenschaftlichen Veröffentlichungen beschreiben 1-Butinyl-2,2,6,6-tetramethylpiperidine, deren Piperidinring in 4-Stellung unsubstituiert ist, und deren mögliche Anwendung als Arzneimittel; so M.E.Zuhair et al., Eur. J. Med. Chem. 27, 93 (1992); J.M.A. Al-Rawi et al. in Org. Magn. Reson. 19, 91 (1982) und Org. Magn. Reson. 15, 285 (1981); B.Karlen et al., J.Med.Chem.13, 651 (1970).

1,6-Bis(2,2,6,6-tetramethylpiperidin-1-yl)-hexadiin, dessen Piperidinringe in 4-Stellung unsubstituiert sind, wird in den Publikationen US-A-3755586; W.B.Lutz et al., J.Org.Chem.27, 1695 (1962); und US-A-3085093 als pharmazeutisch wirksame Verbindung beschrieben. 1,4-Bis(2,2,6,6-tetramethylpiperidin-1-yl)-alkenylene und -alkinylene, deren Piperidinringe in 4-Stellung substituiert sind, werden im US Patent 3,940,363 beschrieben.

Es wurde nun gefunden, daß bestimmte Verbindungen vom Typ 1,4-Diamino-2-butin oder 1,6-Diamino-2,4-hexadiin sich überraschend gut als Stabilisatoren für organisches Material einsetzen lassen.

Gegenstand der Erfindung sind daher zunächst neue Verbindungen der Formel I

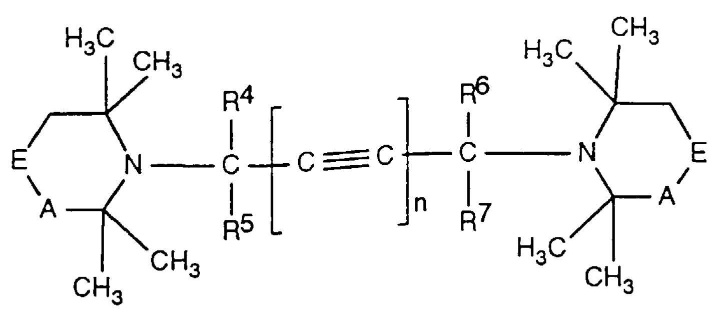

(I)

worin n 1 oder 2 ist, A $-CH_2-$ oder -CO- bedeutet;

wenn A Methylen ist, E die Bedeutung

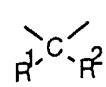

hat, und

wenn A Carbonyl ist, E die Bedeutung -N-$R^3$ hat;

$R^1$ Wasserstoff ist;

$R^2$ -OH; -O-$R^8$; -S-$R^8$; -OCO-$R^8$; -NHCO-$R^8$ oder -N($R^{13}$)$R^{14}$ ist; oder

$R^1$ und $R^2$ gemeinsam einen Substituenten =O bedeuten oder zusammen mit dem Kohlenstoffatom, an das sie gebunden sind, einen Fünf- oder Sechsring einer der Formeln

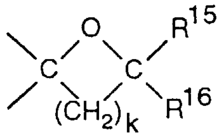, 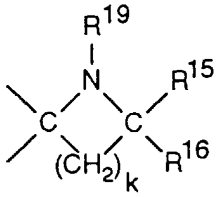, 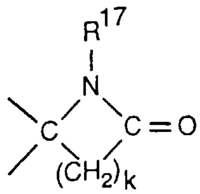, 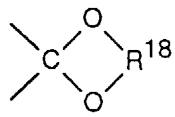, 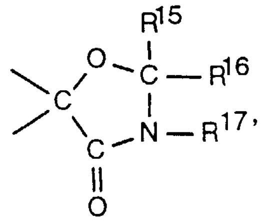,

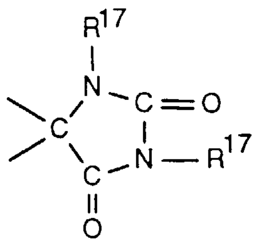

darstellen, wobei k 2 oder 3 ist;

$R^3$ $C_1$-$C_{18}$-Alkyl; $C_3$-$C_{18}$-Alkenyl; $C_5$-$C_8$-Cycloalkyl; Phenyl; Naphthyl;

$C_7$-$C_9$-Phenylalkyl; oder $C_{11}$-$C_{14}$-Naphthylalkyl bedeutet;

$R^4$, $R^5$, $R^6$ und $R^7$, unabhängig voneinander, Wasserstoff oder Methyl darstellen;

$R^8$ $C_1$-$C_{50}$-Alkyl, $C_2$-$C_{18}$-Alkenyl oder Glycidyl bedeutet; oder $C_2$-$C_{50}$-Alkyl bedeutet, welches durch -O-, -S-, -$NR^{19}$- und/oder $C_5$-$C_8$-Cycloalkylen unterbrochen ist; oder $R^8$ $C_5$-$C_{12}$-Cycloalkyl, welches unsubstituiert oder durch 1 bis 4 -$R^{12}$ substituiert ist; $C_6$-$C_{10}$-Aryl, welches unsubstituiert oder durch 1 bis 4 -$R^{12}$ oder -$OR^{12}$ substituiert ist;

oder $C_7$-$C_{50}$-Aralkyl, welches unsubstituiert oder durch $C_5$-$C_8$-Cycloalkyl substituiert und/oder im aliphatischen Teil durch $C_5$-$C_8$-Cycloalkylen unterbrochen und/oder im aliphatischen Teil durch Sauerstoff oder Schwefel unterbrochen und/oder im aromatischen Teil durch 1 bis 4 -$R^{12}$ oder -$OR^{12}$ substituiert ist, bedeutet;

$R^{12}$ $C_1$-$C_{18}$-Alkyl; $C_2$-$C_{18}$-Alkenyl; $C_5$-$C_8$-Cycloalkyl; Phenyl oder Benzyl ist;

$R^{13}$ und $R^{14}$, unabhängig voneinander, jeweils eine der für $R^8$ angegebenen Bedeutungen haben; oder $R^{13}$ und $R^{14}$ zusammen mit dem Stickstoffatom, an das sie gebunden sind, zyklisches Imid der Formel

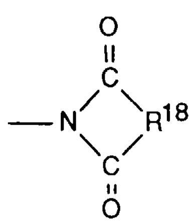

darstellen, dessen Ringstruktur 4 bis 6 Kohlenstoffatome enthält;

$R^{15}$ und $R^{16}$, unabhängig voneinander, H; oder $C_1$-$C_{12}$-Alkyl; oder gemeinsam geradkettiges, α,ω-verknüpftes $C_4$-$C_{13}$-Alkylen bedeuten;

$R^{17}$ H ist oder eine der Bedeutungen von $R^3$ hat;

$R^{18}$ $C_2$-$C_{18}$-Alkylen oder $C_2$-$C_6$-Alkenylen darstellt; und

$R^{19}$ eine der Bedeutungen von $R^3$ hat.

Die erfindungsgemäßen Verbindungen lassen sich vorteilhaft zur Stabilisierung organischen Materials gegen den schädigenden Einfluß von Licht, Sauerstoff und/oder Wärme einsetzen. Verbindungen der Formel I, worin A Methylen ist und E die Bedeutung -CH(OH)- hat, stellen darüberhinaus auch wertvolle Ausgangsstoffe für die Herstellung weiterer Stabilisatoren dar; sie bilden daher für sich einen bevorzugten Gegenstand der Erfindung.

A ist vorzugsweise Methylen, E hat vorzugsweise die Bedeutung

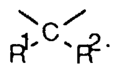

Besonders bevorzugt sind Verbindungen der Formel I, worin n 1 ist.

Treten innerhalb einer Formel mehrere gleich benannte Reste auf, so können diese gleich oder verschieden sein und jeder dieser Reste hat, unabhängig von dem oder den anderen, eine der angegebenen Bedeutungen. Beispielsweise umfassen $R^1$ und $R^2$ in der gemeinsamen Bedeutung

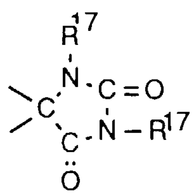 sowohl 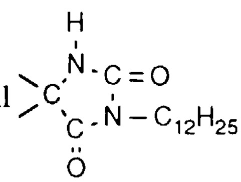

als auch

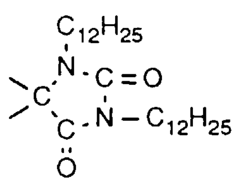 und 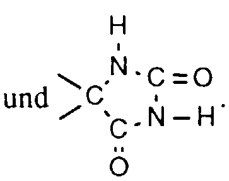

Bevorzugt sind Verbindungen der Formel I, worin $R^4$ und $R^6$ Wasserstoff sind; besonders bevorzugt sind alle 4 Reste $R^4$, $R^5$, $R^6$ und $R^7$ Wasserstoff.

$R^8$, $R^{13}$ oder $R^{14}$ als Aryl bedeuten einen aromatischen Kohlenwasserstoffrest wie beispielsweise Phenyl oder Naphthyl. Aralkyl bedeutet Alkyl, das durch einen aromatischen Kohlenwasserstoffrest, z.B. einen $C_6$-$C_{10}$-Kohlenwasserstoffrest, substituiert ist; Beispiele dafür sind neben anderen Benzyl oder α-Methylbenzyl. Die angegebenen C-Zahlen (Indices) bezeichnen die Gesamtzahl an Kohlenstoffatomen im angegebenen Rest.

$R^8$, $R^{13}$ und $R^{14}$ umfassen beispielsweise folgende Bedeutungen:
Methyl, Ethyl, Propyl, Isopropyl, n-Butyl, sec-Butyl, Isobutyl, t-Butyl, 2-Ethylbutyl, n-Pentyl, Isopentyl, 1-Methylpentyl, 1,3-Dimethylbutyl, n-Hexyl, 1-Methylhexyl, n-Heptyl, Isoheptyl, 1,1,3,3-Tetramethylbutyl, 1-Methylheptyl, 3-Methylheptyl, n-Octyl, 2-Ethylhexyl, 1,1,3-Trimethylhexyl, 1,1,3,3-Tetramethylpentyl, Nonyl, Decyl, Undecyl, 1-Methylundecyl, Dodecyl, 1,1,3,3,5,5-Hexamethylhexyl, Tridecyl, Tetradecyl, Pentadecyl, Hexadecyl, Heptadecyl, Octadecyl, Eicosyl, Docosyl, Pentakosyl, Triakosyl, Tetrakontyl;
$-C_2H_4-O-C_2H_4-O-C_{12}H_{25}$, $-(C_2H_4-O)_4-C_4H_9$, $-(C_2H_4-O)_6-C_4H_9$, $-(C_2H_4-O)_4-C_2H_5$, $-(C_2H_4-O)_6-C_2H_5$;
Cyclopentyl, Cyclohexyl, Cycloheptyl, Cyclooctyl, 2- oder 4-Methylcyclohexyl, Dimethylcyclohexyl, Trimethylcyclohexyl, t-Butylcyclohexyl;
$C_6$-$C_{10}$-Aryl wie Phenyl oder Naphthyl; mit $C_1$-$C_4$-Alkyl substituiertes Phenyl;
Arylalkyl oder substituiertes Arylalkyl wie Benzyl, Phenethyl, 3-Phenylpropyl, α-Methylbenzyl, α,α-Dimethylbenzyl, $-C_2H_4-O-C_2H_4-O-C_{12}H_{24}-C_6H_5$, $-(C_2H_4-O)_4-CH_2-C_6H_5$, $-(C_2H_4-O)_6-C_4H_8-C_6H_5$, $-CH_2-C_6H_4-C(CH_3)_2-C_6H_5$.

Bevorzugte Bedeutungen von $R^3$, $R^{17}$ und $R^{19}$ sind $C_1$-$C_{18}$-Alkyl; $C_3$-$C_5$-Alkenyl; Cyclohexyl; Benzyl und α-Methylbenzyl. Für $R^{17}$ ist zusätzlich Wasserstoff eine bevorzugte Bedeutung.

Enthalten die Reste $R^8$, $R^{13}$ oder $R^{14}$ Alkyl, welches durch -O-, -S- oder $-NR^{19}-$ unterbrochen ist, so handelt

es sich um Alkyl mit mindestens 2, vorzugsweise mindestens 4 Kohlenstoffatomen, das bevorzugt durch 1-6 Gruppen -O-, -S- oder -$NR^{19}$-, besonders durch 1-6 -O- unterbrochen ist; die Heteroatome binden vorzugsweise an Kohlenstoffatome und nicht an andere Heteroatome, Strukturen der Typen -O-O- oder -$NR^{19}$-$NR^{19}$- treten nicht auf. Besonders bevorzugt stellen diese Reste Polyoxyethylenketten dar, deren Enden mit $C_1$-$C_8$-Alkyl abgesättigt sind.

$R^8$, $R^{13}$ und $R^{14}$ stellen besonders bevorzugt langkettige und/oder sperrige Reste dar, beispielsweise $C_6$-$C_{36}$-Alkyl; durch -O- unterbrochenes $C_6$-$C_{36}$-Alkyl; $C_5$-$C_9$-Cycloalkyl; Phenyl; $C_7$-$C_{36}$-Phenylalkyl; oder $C_7$-$C_{36}$-Phenylalkyl, welches im aliphatischen Teil durch -O- unterbrochen ist; besonders bevorzugt ist $C_6$-$C_{18}$-Alkyl oder $C_7$-$C_9$-Phenylalkyl.

$R^3$, $R^8$, $R^{12}$, $R^{13}$, $R^{14}$, $R^{17}$ und $R^{19}$ als Alkenyl sind im Rahmen ihrer angegebenen Bedeutungen beispielsweise Ethenyl, 1- oder 2-Propenyl, Butenyl, Pentenyl, Hexenyl, Heptenyl, Octenyl, Nonenyl, Decenyl, Undecenyl, Dodecenyl, Tridecenyl, Tetradecenyl, Pentadecenyl, Hexadecenyl, Heptadecenyl oder Octadecenyl. $R^3$, $R^{17}$ und $R^{19}$ als Alkenyl sind vorzugsweise Allyl; $R^8$, $R^{12}$, $R^{13}$ und $R^{14}$ als Alkenyl sind vorzugsweise Vinyl, $\alpha$-Methylvinyl oder Allyl.

$R^3$, $R^{12}$, $R^{17}$ und $R^{19}$ als Alkyl sind beispielsweise Methyl, Ethyl, Propyl, Isopropyl, Butyl, Isobutyl, tert-Butyl, Pentyl, Hexyl, Heptyl, Octyl, Nonyl, Decyl, Undecyl, Dodecyl, Tridecyl, Tetradecyl, Pentadecyl, Hexadecyl, Heptadecyl oder Octadecyl; als Cycloalkyl Cyclopentyl, Cyclohexyl, Cycloheptyl, Cyclooctyl, besonders Cyclohexyl.

Halogen bezeichnet ein Atom aus der Reihe F, Cl, Br, I; meist steht es für Cl oder Br, vor allem für Cl.

Unter den Verbindungen der Formel I sind diejenigen von hervorgehobenem Interesse, worin A Methylen ist, E die Bedeutung

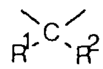

hat, und

$R^2$-OH; -O-$R^8$; -OCO-$R^8$; -NHCO-$R^8$ oder -N($R^{13}$)$R^{14}$ ist; oder

$R^1$ und $R^2$ gemeinsam einen Substituenten =O bedeuten oder zusammen mit dem Kohlenstoffatom, an das sie gebunden sind, einen Fünf- oder Sechsring einer der Formeln

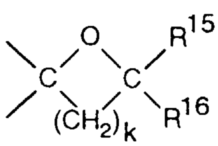 , 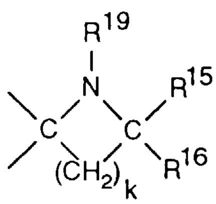 , 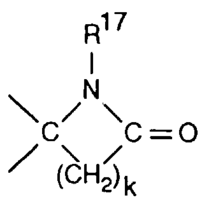 , 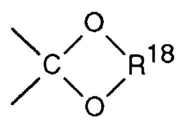 , 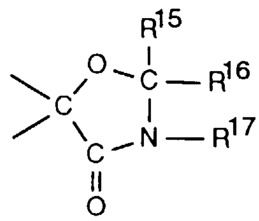 ,

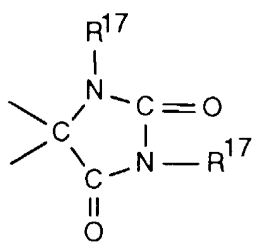

darstellen, wobei k 2 oder 3 ist;

$R^4$, $R^5$, $R^6$ und $R^7$ Wasserstoff sind,

$R^8$ $C_1$-$C_{50}$-Alkyl, $C_2$-$C_{18}$-Alkenyl oder Glycidyl bedeutet; oder $C_2$-$C_{50}$-Alkyl bedeutet, welches durch -O-, -S-, -$NR^{19}$- und/oder $C_5$-$C_8$-Cycloalkylen unterbrochen ist; oder $R^8$ $C_5$-$C_{12}$-Cycloalkyl, welches unsubstituiert oder durch 1 bis 4 -$R^{12}$ substituiert ist; Phenyl, welches unsubstituiert oder durch 1 bis 4 -$R^{12}$ oder -$OR^{12}$ substituiert ist; oder $C_7$-$C_{18}$-Aralkyl, welches unsubstituiert oder im aromatischen Teil durch 1 bis 4 -$R^{12}$ oder -$OR^{12}$ substituiert ist, bedeutet;

$R^{12}$ $C_1$-$C_{18}$-Alkyl; $C_2$-$C_{18}$-Alkenyl; $C_5$-$C_8$-Cycloalkyl; Phenyl oder Benzyl ist;

$R^{13}$ und $R^{14}$, unabhängig voneinander, jeweils eine der für $R^8$ angegebenen Bedeutungen haben; oder

$R^{13}$ und $R^{14}$ zusammen mit dem Stickstoffatom, an das sie gebunden sind, zyklisches Imid der Formel

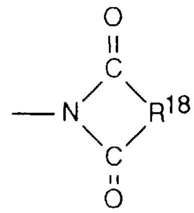

darstellen, dessen Ringstruktur 4 bis 6 Kohlenstoffatome enthält; und
$R^{18}$ $C_2$-$C_{18}$-Alkylen oder $C_2$-$C_6$-Alkenylen darstellt.

Bevorzugt sind darunter Verbindungen der Formel I, worin A Methylen ist, E die Bedeutung

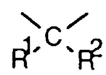

hat, und
$R^2$ -OH; -O-$R^8$; -OCO-$R^8$; -NHCO-$R^8$ oder -N($R^{13}$)$R^{14}$ ist; oder
$R^1$ und $R^2$ zusammen mit dem Kohlenstoffatom, an das sie gebunden sind, einen Fünf- oder Sechsring der Formel

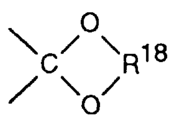 oder 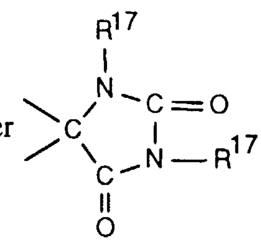

darstellen;
$R^4$, $R^5$, $R^6$ und $R^7$ Wasserstoff sind,
$R^8$ $C_1$-$C_{36}$-Alkyl, $C_2$-$C_8$-Alkenyl oder Glycidyl bedeutet; oder $C_2$-$C_{36}$-Alkyl bedeutet, welches durch -O-, -$NR^{19}$- und/oder $C_5$-$C_8$-Cycloalkylen unterbrochen ist; oder $R^8$ $C_5$-$C_{12}$-Cycloalkyl; Phenyl, welches unsubstituiert oder durch -$R^{12}$ oder -$OR^{12}$ substituiert ist; oder $C_7$-$C_9$-Aralkyl, welches unsubstituiert oder im aromatischen Teil durch -$R^{12}$ substituiert ist, bedeutet;
$R^{12}$ $C_1$-$C_8$-Alkyl; $C_2$-$C_8$-Alkenyl; Cyclohexyl; Phenyl oder Benzyl ist;
$R^{13}$ und $R^{14}$, unabhängig voneinander, jeweils eine der für $R^8$ angegebenen Bedeutungen haben; oder
$R^{13}$ und $R^{14}$ zusammen mit dem Stickstoffatom, an das sie gebunden sind, zyklisches Imid der Formel

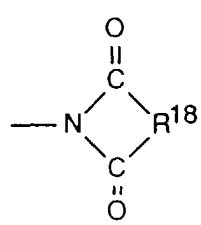

darstellen, dessen Ringstruktur 4 bis 6 Kohlenstoffatome enthält; und
$R^{18}$ $C_2$-$C_3$-Alkylen oder $C_2$-$C_3$-Alkenylen darstellt;
vor allem solche Verbindungen, worin A Methylen ist, E die Bedeutung

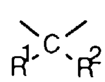

hat, und
$R^2$ -OH; -O-$R^8$; -OCO-$R^8$; -NHCO-$R^8$ oder-N($R^{13}$)$R^{14}$ ist; oder
$R^1$ und $R^2$ zusammen mit dem Kohlenstoffatom, an das sie gebunden sind, einen Fünf- oder Sechsring der Formel

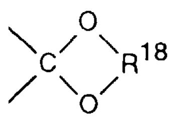 oder 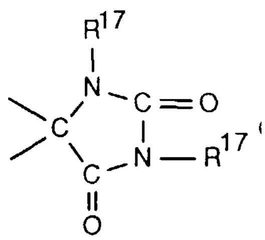

darstellen;
$R^4$, $R^5$, $R^6$ und $R^7$ Wasserstoff sind,
$R^8$ $C_1$-$C_{18}$-Alkyl, $C_2$-$C_8$-Alkenyl oder Glycidyl bedeutet; oder $R^8$ $C_5$-$C_{12}$-Cycloalkyl; Phenyl, welches unsubstituiert oder durch -$R^{12}$ substituiert ist; oder $C_7$-$C_9$-Aralkyl bedeutet;
$R^{12}$ $C_1$-$C_8$-Alkyl; Vinyl; $\alpha$-Methylvinyl oder Allyl ist;
$R^{13}$ und $R^{14}$, unabhängig voneinander, jeweils eine der für $R^8$ angegebenen Bedeutungen haben.

Von besonderem technischem Interesse sind solche Verbindungen der Formel I, worin n 1 oder 2 ist, A Methylen ist, E die Bedeutung

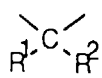

hat, und
$R^2$ -OH; $C_1$-$C_{18}$-Alkoxy; Allyloxy; $C_7$-$C_9$-Aralkoxy; Glycidyloxy; $C_2$-$C_{18}$-Alkanoyloxy; $C_3$-$C_4$-Alkenoyloxy; Benzoyloxy; Formamidyl; $C_2$-$C_{18}$-Alkanoylamido; Benzoylamido; oder -N($R^{13}$)$R^{14}$ ist; oder
$R^1$ und $R^2$ zusammen mit dem Kohlenstoffatom, an das sie gebunden sind, einen Fünf- oder Sechsring der Formel

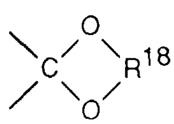 oder 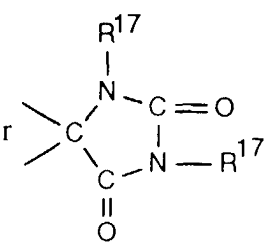

darstellen;
$R^4$, $R^5$, $R^6$ und $R^7$ Wasserstoff sind;
$R^{13}$ und $R^{14}$, unabhängig voneinander, $C_1$-$C_{18}$-Alkyl bedeuten; und $R^{18}$ $C_2$-$C_3$-Alkylen oder $C_2$-Alkenylen darstellt;
vor allem solche, worin A Methylen ist, E die Bedeutung

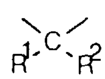

hat, und
$R^2$ -OH; $C_6$-$C_{18}$-Alkoxy; Allyloxy; Benzyloxy; Glycidyloxy; $C_2$-$C_{18}$-Alkanoyloxy; oder $C_3$-$C_4$-Alkenoyloxy ist; oder
$R^1$ und $R^2$ zusammen mit dem Kohlenstoffatom, an das sie gebunden sind, einen Fünfring der Formel

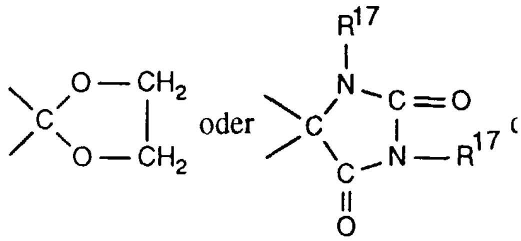

darstellen; und

$R^4$, $R^5$, $R^6$ und $R^7$ Wasserstoff sind.

Darunter ist eine Verbindung der Formel I von besonderer Bedeutung, worin A Methylen ist und E die Bedeutung

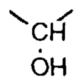

hat, beispielsweise die Verbindung

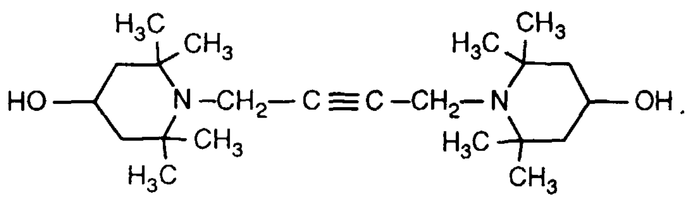

Die neuen Verbindungen der Formel I können in Analogie zu bekannten Herstellungsverfahren erhalten werden, wie sie beispielsweise in den Publikationen US-A-3085093; J. Org. Chem. 27, 1695 (1962); Eur. J. Med. Chem. 27, 93 (1992); US-A-4386127; oder J. Med. Chem. 13, 651 (1970); und in der dort zitierten Literatur beschrieben sind. So können 2,2,6,6-Tetramethylpiperidine mit freier 1-Stellung mit Butinylhalogeniden oder -dihalogeniden oder zunächst mit Propargylhalogenid umgesetzt werden. Als Halogenide werden häufig die Chloride und insbesondere die Bromide eingesetzt. Erhaltene 1-Propargyl-2,2,6,6-tetramethylpiperidine können mit Formaldehyd und weiterem 2,2,6,6-Tetramethylpiperidin zu den disubstituierten Butinen umgesetzt, oder mit Hilfe eines Katalysators zum entsprechenden Hexadiin dimerisiert werden.

Bevorzugt werden Verbindungen der Formel I jedoch gemäß dem folgenden, neuen Verfahren hergestellt.

Danach lassen sich Verbindungen der Formel I erhalten, indem ein Ester einer aromatischen Sulfonsäure der Formel II

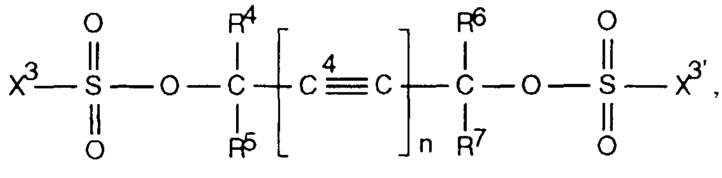
(II)

worin $X^3$ und $X^{3'}$, unabhängig voneinander, Phenyl oder durch $C_1$-$C_8$-Alkyl, $C_1$-$C_8$-Alkoxy oder Halogen substituiertes Phenyl sind,

mit einer Verbindung der Formel III

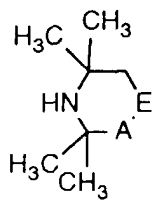

(III)

umgesetzt wird. Dieses Verfahren stellt ebenfalls einen Gegenstand der Erfindung dar.

$X^3$ und $X^{3'}$ sind vorzugsweise Phenyl, Tolyl, Chlorphenyl oder Methoxyphenyl; insbesondere Phenyl oder Tolyl. Vorzugsweise sind $X^3$ und $X^{3'}$ gleich. Besonders bevorzugt werden im erfindungsgemäßen Verfahren als Edukt der Formel II 2-Butin-1,4-diol-ditosylat oder 2,4-Hexadiin-1,6-diol-ditosylat eingesetzt, vor allem 2-Butin-1,4-diolditosylat.

Allgemein werden etwa 2 Äquivalente der Verbindung der Formel III pro Äquivalent der Verbindung der Formel II eingesetzt. Vorteilhaft kann die Verbindung der Formel III auch im Überschuß verwendet werden, beispielsweise in einer Menge von 1,5-4, besonders 2-3 Äquivalenten der Verbindung der Formel III pro Sulfonyloxygruppe in Verbindung der Formel II; das nicht umgesetzte Edukt kann in diesem Fall vom Produkt abgetrennt und erneut in die Reaktion eingesetzt werden.

Die Umsetzung erfolgt zweckmäßig in einem inerten Lösungsmittel. Als Lösungsmittel werden meist polare organische Lösungsmittel eingesetzt werden wie z.B. Alkohole, halogenierte Kohlenwasserstoffe, Ester, Ether, Ketone, Amide, Nitrile, tert. Amine oder Sulfoxide; geeignet sind beispielsweise Methylethylketon, tert.-Butanol, Dimethylformamid, Tetrahydrofuran, Dioxan, Chloroform, Pyridin, Dibutylether, Dimethylsulfoxid, Acetonitril; besonders bevorzugt ist Acetonitril.

Die Temperatur kann während der Reaktion im Bereich von 0 bis 200°C liegen, zweckmäßig beträgt die Temperatur 20-160°C, vor allem 40-140°C.

Die Temperatur der Reaktionsmischung kann für die Dauer der Reaktion im Siedebereich gehalten werden (Rückfluß). Dazu wird eine Lösemittel enthaltende Reaktionsmischung, im allgemeinen unter Normaldruck, zum Siedepunkt erwärmt und das verdampfte Lösemittel mit Hilfe eines geeigneten Kühlers kondensiert und wieder der Reaktionsmischung zugeführt.

Die Reaktion kann unter Ausschluß von Sauerstoff durchgeführt werden, beispielsweise durch Spülen mit einem Inertgas wie Argon; Sauerstoff stört jedoch nicht in jedem Fall, so daß die Reaktion auch ohne die genannte Maßnahme durchgeführt werden kann.

Nach beendeter Reaktion kann die Aufarbeitung nach gängigen Methoden erfolgen; zweckmäßig wird die Mischung zunächst mit Wasser verdünnt, beispielsweise indem das Reaktionsgemisch in das 1-4-fache Volumen Eiswasser gegeben wird; anschließend kann das Produkt direkt abgetrennt oder extrahiert werden, zur Extraktion eignet sich z.B. Essigester oder Chloroform. Wird extrahiert, so kann das Produkt in üblicher Weise durch Entfernen des Lösemittels isoliert werden; dies geschieht zweckmäßig nach Trocknen der organischen Phase. Möglich sind auch das Einschalten weiterer Reinigungsschritte wie beispielsweise Waschen mit wässriger NaCl-Lösung, Dispergieren von Aktivkohle, Filtrieren, Umkristallisieren und/oder Destillieren.

Verbindungen der Formel II sind zum Teil bekannt; ihre Herstellung kann beispielsweise gemäß Angew. Chem. 104, 1652-1654 (1992) oder in Analogie zu dem dort beschriebenen Verfahren erfolgen. Verbindungen der Formel III sind bekannt und zum Teil kommerziell erhältlich.

Die gemäß den oben beschriebenen Herstellungsverfahren erhaltenen Verbindungen können als solche verwendet werden; sie können jedoch auch nach bekannten Methoden bzw. in Analogie zu beschriebenen Verfahren weiter derivatisiert werden. Als Beispiel sei hier nur die weitere Umsetzung eines Diols der Formel I genannt, worin E die Gruppe -CH(OH)- darstellt, das sich nach gängigen Methoden beispielsweise in entsprechende Ether, Ester oder, unter Austausch von Sauerstoff gegen Stickstoff, in entsprechende Amine überführen läßt.

Die Verbindungen der Formel I eignen sich zum Stabilisieren von organischen Materialien gegen thermischen, oxidativen und aktinischen Abbau.

Beispiele für derartige Materialien sind:

1. Polymere von Mono- und Diolefinen, beispielsweise Polypropylen, Polyisobutylen, Polybuten-1, Poly-4-methylpenten-1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen niederer Dichte (LDPE), lineares Polyethylen niederer Dichte (LLDPE), verzweigtes Polyethylen niederer Dichte (VLDPE).

Polyolefine, d.h. Polymere von Monoolefinen, wie sie beispielhaft im vorstehenden Absatz erwähnt sind, insbesondere Polyethylen und Polypropylen, können nach verschiedenen Verfahren hergestellt werden, insbesondere nach den folgenden Methoden:

a) radikalisch (gewöhnlich bei hohem Druck und hoher Temperatur).

b) mittels Katalysator, wobei der Katalysator gewöhnlich ein oder mehrere Metalle der Gruppe IVb, Vb, VIb oder VIII enthält. Diese Metalle besitzen gewöhnlich einen oder mehrere Liganden wie Oxide, Halogenide, Alkoholate, Ester, Ether, Amine, Alkyle, Alkenyle und/oder Aryle, die entweder $\pi$- oder $\sigma$-koordiniert sein können. Diese Metallkomplexe können frei oder auf Träger fixiert sein, wie beispielsweise auf aktiviertem Magnesiumchlorid, Titan(III)chlorid, Aluminiumoxid oder Siliziumoxid. Diese Katalysatoren können im Polymerisationsmedium löslich oder unlöslich sein. Die Katalysatoren können als solche in der Polymerisation aktiv sein, oder es können weitere Aktivatoren verwendet werden, wie beispielsweise Metallalkyle, Metallhydride, Metallalkylhalogenide, Metallalkyloxide oder Metallalkyloxane, wobei die Metalle Elemente der Gruppen Ia, IIa und/oder IIIa sind. Die Aktivatoren können beipielsweise mit weiteren Ester-, Ether-, Amin- oder Silylether-Gruppen modifiziert sein. Diese Katalysatorsysteme werden gewöhnlich als Phillips, Standard Oil Indiana, Ziegler (-Natta), TNZ (DuPont), Metallocen oder Single Site Katalysatoren (SSC) bezeichnet.

2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).

3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat- Copolymere, Ethylen-Vinylacetat-Copolymere und deren Copolymere mit Kohlenstoffmonoxid, oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere, LLDPE/Ethylen-Acrylsäure -Copolymere und alternierend oder statistisch aufgebaute Polyalkylen/Kohlenstoffmonoxid-Copolymere und deren Mischungen mit anderen Polymeren wie z.B. Polyamiden.

4. Kohlenwasserstoffharze (z.B. $C_5$-$C_9$) inklusive hydrierte Modifikationen davon (z.B.oKlebrigmacherharze) und Mischungen von Polyalkylenen und Stärke.

5. Polystyrol, Poly-(p-methylstyrol), Poly-($\alpha$-methylstyrol).

6. Copolymere von Styrol oder $\alpha$-Methylstyrol mit Dienen oder Acrylderivaten, wie z.B. Styrol-Butadien, Styrol-Acrylnitril, Styrol-Alkylmethacrylat, Styrol-Butadien-Alkylacrylat und -methacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.

7. Pfropfcopolymere von Styrol oder $\alpha$-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 6) genannten Copolymeren, wie sie z.B. als sogenannte ABS-, MBS-, ASA- oder AES-Polymere bekannt sind.

8. Halogenhaltige Polymere, wie z.B. Polychloropren, Chlorkautschuk, chloriertes oder chlorsulfoniertes Polyethylen, Copolymere von Ethylen und chloriertem Ethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat

9. Polymere, die sich von a,β-ungesättigten Säuren und deren Derivaten ableiten, wie Polyacrylate und Polymethacrylate, mit Butylacrylat schlagzäh modifizierte Polymethylmethacrylate, Polyacrylamide und Polyacrylnitrile.

10. Copolymere der unter 9) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-VinylhalogenidCopolymere oder Acrylnitril-Alkylmethacrylat-Butadien-Terpolymere.

11. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.

12. Homo- und Copolymere von cyclischen Ethern, wie Polyalkylenglykole, Polyethylenoxyd, Polypropylenoxyd oder deren Copolymere mit Bisglycidylethern.

13. Polyacetale, wie Polyoxymethylen, sowie solche Polyoxymethylene, die Comonomere, wie z.B. Ethylenoxid, enthalten; Polyacetale, die mit thermoplastischen Polyurethanen, Acrylaten oder MBS modifiziert sind.

14. Polyphenylenoxide und -sulfide und deren Mischungen mit Styrolpolymeren oder Polyamiden.

15. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.

16. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, 12/12, Polyamid 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid oder Poly-m-phenylen-isophthalamid. Block-Copolymere der vorstehend genanntenPolyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").

17. Polyharnstoffe, Polyimide, Polyamid-imide und Polybenzimidazole.

18. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.

19. Polycarbonate und Polyestercarbonate.

20. Polysulfone, Polyethersulfone und Polyetherketone.

21. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

22. Trocknende und nicht-trocknende Alkydharze.

23. Ungesättigte Polyesterharze, die sich von Copolyestern gsättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

24. Vernetzbare Acrylharze, die sich von substituierten Acrylsäureestern ableiten, wie z.B. von Epoxyacrylaten,

Urethan-acrylaten oder Polyester-acrylaten.

25. Alkydharze, Polyesterharze und Acrylatharze, die mit Melaminharzen, Harnstoffharzen, Isocyanaten, Isocyanuraten, Polyisocyanaten oder Epoxidharzen vernetzt sind.

26. Vernetzte Epoxidharze, die sich von Polyepoxiden ableiten, z.B. von Bis-glycidylethern oder von cycloaliphatischen Diepoxiden.

27. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.

28. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO.

Weitere Gegenstände der Erfindung sind daher Zusammensetzungen enthaltend (a) ein gegen oxidativen, thermischen oder/und aktinischen Abbau empfindliches organisches Material und (b) mindestens eine Verbindung der Formel I, sowie die Verwendung von Verbindungen der Formel I zum Stabilisieren von organischem Material gegen oxidativen, thermischen oder aktinischen Abbau.
Die Erfindung umfaßt ebenfalls ein Verfahren zum Stabilisieren von organischem Material gegen thermischen, oxidativen oder/und aktinischen Abbau, dadurch gekennzeichnet, daß man diesem Material mindestens eine Verbindung der Formel I zusetzt.

Von besonderem Interesse ist die Verwendung von Verbindungen der Formel I als Stabilisatoren in synthetischen organischen Polymeren sowie entsprechende Zusammensetzungen.

Vorzugsweise handelt es sich bei den zu schützenden organischen Materialien um natürliche, halbsynthetische oder bevorzugt synthetische organische Materialien. Besonders bevorzugt sind synthetische organische Polymere oder Gemische solcher Polymere, insbesondere thermoplastische Polymere wie Polyolefine, vor allem Polyethylen und Polypropylen (PP). Ebenfalls besonders bevorzugte organische Materialien sind Überzugszusammensetzungen. Unter photographischen Materialien sind insbesondere die Materialien zu verstehen, die in Research Disclosure 1990, 31429 (Seiten 474-480) für die photographische Reproduktion und andere Reproduktionstechniken beschrieben sind. Im Sinne der Erfindung vorteilhaft zu stabilisierende Überzugszusammensetzungen sind beispielsweise beschrieben in Ullmann's Encyclopedia of Industrial Chemistry, 5. Ed., Vol. A18, pp. 359-464, VCH Verlagsgesellschaft, Weinheim 1991.

Allgemein werden die Verbindungen der Formel I dem zu stabilisierenden Material in Mengen von 0,01 bis 10 %, bevorzugt 0,01 bis 5 %, insbesondere 0,01 bis 2 %, bezogen auf das Gesamtgewicht der stabilisierten Zusammensetzung, zugesetzt. Besonders bevorzugt ist der Einsatz der erfindungsgemäßen Verbindungen in Mengen von 0,05 bis 1,5 %, vor allem 0,1 bis 0,5 %.

Die Einarbeitung in die Materialien kann beispielsweise durch Einmischen oder Aufbringen der Verbindungen der Formel I und gegebenenfalls weiterer Additive nach den in der Technik üblichen Methoden erfolgen. Handelt es sich um Polymere, insbesondere synthetische Polymere, kann die Einarbeitung vor oder während der Formgebung, oder durch Aufbringen der gelösten oder dispergierten Verbindung auf das Polymere, gegebenenfalls unter nachträglichem Verdunsten des Lösungsmittels erfolgen. Im Fall von Elastomeren können diese auch als Latices stabilisiert werden. Eine weitere Möglichkeit der Einarbeitung der Verbindungen der Formel I in Polymere besteht in deren Zugabe vor, während oder unmittelbar nach der Polymerisation der entsprechenden Monomeren bzw. vor der Vernetzung. Die Verbindung der Formel I kann dabei als solche, aber auch in encapsulierter Form (z.B in Wachsen, Ölen oder Polymeren) zugesetzt werden. Im Falle der Zugabe vor oder während der Polymerisation können die Verbindungen der Formel I auch als Regulator für die Kettenlänge der Polymere (Kettenabbrecher) wirken.

Die Verbindungen der Formel I können auch in Form eines Masterbatches, der diese Verbindung beispielsweise in einer Konzentration von 2,5 bis 25 Gew.-% enthält, den zu stabilisierenden Kunststoffen zugesetzt werden.

Zweckmäßig kann die Einarbeitung der Verbindungen der Formel I nach folgenden Methoden erfolgen:

- als Emulsion oder Dispersion (z.B. zu Latices oder Emulsionspolymeren),
- als Trockenmischung während des Vermischens von Zusatzkomponenten oder Polymermischungen,
- durch direktes Zugeben in die Verarbeitungsapparatur (z.B. Extruder, Innenmischer usw.),
- als Lösung oder Schmelze.

Erfindungsgemäße Polymerzusammensetzungen können in verschiedener Form angewendet bzw. zu verschiedenen Produkten verarbeitet werden, z.B. als (zu) Folien, Fasern, Bändchen, Formmassen, Profilen, oder als Bindemittel für Lacke, Klebstoffe oder Kitte.

Neben den Verbindungen der Formel I können die erfindungsgemäßen Zusammensetzungen als zusätzliche Komponente (c) ein oder mehrere herkömmliche Additive enthalten, wie beispielsweise die unten angegebenen.

Die herkömmlichen Additive werden zweckmäßig in Mengen von 0,1-10, beispielsweise 0,2-5 Gew.-%, bezogen auf das zu stabilisierende Polymer, eingesetzt.

1. Antioxidantien

1.1. Alkylierte Monophenole, z.B. 2,6-Di-tert-butyl-4-methylphenol, 2-butyl-4,6-di-methylphenol, 2,6-Di-tert-butyl-4-ethylphenol, 2,6-Di-tert-butyl-4-n-butylphenol, 2,6-Di-tert-butyl-4-iso-butylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tert-butyl-4-methoxymethylphenol, 2,6-Di-nonyl-4-methylphenol, 2,4-Dimethyl-6-(1'-methyl-undec-1'-yl)-phenol, 2,4-Dimethyl-6-(1'-methyl-heptadec-1'-yl)-phenol, 2,4-Dimethyl-6-(1'-methyl-tridec-1'-yl)-phenol und Mischungen davon.

1.2. Alkylthiomethylphenole, z.B. 2,4-Di-octylthiomethyl-6-tert-butylphenol, 2,4-Di-octylthiomethyl-6-methylphenol, 2,4-Di-octylthiomethyl-6-ethylphenol, 2,6-Di-dodecylthiomethyl-4-nonylphenol.

1.3.Hydrochinone und alkylierte Hydrochinone, z. B. 2,6-Di-tert-butyl-4-methoxyphenol, 2,5-Di-tert-butyl-hydrochinon, 2,5-Di-tert-amyl-hydrochinon, 2,6-Diphenyl-4-octadecyloxyphenol, 2,6-Di-tert-butyl-hydrochinon, 2,5-Di-tert-butyl-4-hydroxyanisol, 3,5-Di-tert-butyl-4-hydroxyanisol, 3,5-Di-tert-butyl-4-hydroxyphenyl-stearat, Bis-(3,5-di-tert-butyl-4-hydroxyphenyl)adipat.

1.4. Tocopherole, z.B. α-Tocopherol, β-Tocopherol, γ-Tocopherol, δ-Tocopherol und Mischungen davon (Vitamin E).

1.5.Hydroxylierte Thiodiphenylether, z.B. 2,2'-Thio-bis-(6-tert-butyl-4-methylphenol), 2,2'-Thio-bis-(4-octylphenol), 4,4'-Thio-bis-(6-tert-butyl-3-methylphenol), 4,4'-Thio-bis-(6-tert-butyl-2-methylphenol), 4,4'-Thio-bis-(3,6-di-sec.-amylphenol), 4,4'-Bis-(2,6-dimethyl-4-hydroxyphenyl)-disulfid.

1.6. Alkyliden-Bisphenole, z.B. 2,2'-Methylen-bis-(6-tert-butyl-4-methylphenol), 2,2'-Methylen-bis-(6-tert-butyl-4-ethylphenol), 2,2'-Methylen-bis-[4-methyl-6-(α-methylcyclohexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-tert-butylphenol), 2,2'-Ethyliden-bis-(4,6-di-tert-butylphenol), 2,2'-Ethyliden-bis-(6-tert-butyl-4-isobutylphenol), 2,2'-Methylen-bis-[6-(α-methylbenzyl)-4-nonylphenol], 2,2'-Methylen-bis-[6-(α,α-dimethylbenzyl)-4-nonylphenol], 4,4'-Methylen-bis-(2,6-di-tert-butylphenol), 4,4'-Methylen-bis-(6-tert-butyl-2-methylphenol), 1,1-Bis-(5-tert-butyl-4-hydroxy-2-methylphenyl)-butan, 2,6-Bis-(3-tert-butyl-5-methyl-2-hydroxybenzyl)-4-methylphenol, 1,1,3-Tris-(5-tert-butyl-4-hydroxy-2-methylphenyl)-butan, 1,1-Bis-(5-tert-butyl-4-hydroxy-2-methylphenyl)-3-n-dodecylmercaptobutan, Ethylenglycol-bis-[3,3-bis-(3'-tert-butyl-4'-hydroxyphenyl)-butyrat], Bis-(3-tert-butyl-4-hydroxy-5-methyl-phenyl)-dicyclopentadien, Bis-[2-(3'-tert-butyl-2'-hydroxy-(5'-methyl-benzyl)-6-tert-butyl-4-methyl-phenyl]-terephthalat, 1,1-Bis-(3,5-dimethyl-2-hydroxyphenyl)-butan, 2,2-Bis-(3,5-di-tert-butyl-4-hydroxyphenyl)-propan, 2,2-Bis-(5-tert-butyl-4-hydroxy-2-methylphenyl)-4-n-dodecylmercaptobutan, 1,1,5,5-Tetra-(5-tert-butyl-4-hydroxy-2-methylphenyl)-pentan.

1.7. O-, N- und S-Benzylverbindungen, z. B. 3,5,3',5'-Tetra-tert-butyl-4,4'-dihydroxydibenzylether, Octadecyl-4-hydroxy-3,5-dimethylbenzyl-mercaptoacetat, Tris-(3,5-di-tert-butyl-4-hydroxybenzyl)-amin, Bis-(4-tert-butyl-3-hydroxy-2,6-dimethylbenzyl)-dithioterephthalat, Bis-(3,5-di-tert-butyl-4-hydroxybenzyl)-sulfid, Isooctyl-3,5-di-tert-butyl-4-hydroxybenzyl-mercaptoacetat.

1.8. Hydroxybenzylierte Malonate, z.B. Dioctadecyl-2,2-bis-(3,5-di-tert-butyl-2-hydroxybenzyl)-malonat, Di-octadecyl-2-(3-tert-butyl-4-hydroxy-5-methylbenzyl)-malonat, Di-dodecylmercaptoethyl-2,2-bis-(3,5-di-tert-butyl4-hydroxybenzyl)-malonat, Di-[4-(1,1,3,3,3-tetramethylbutyl)-phenyl]-2,2-bis-(3,5-di-tert-butyl-4-hydroxybenzyl)-malonat.

1.9. Hydroxybenzyl-Aromaten, z.B. 1,3,5-Tris-(3,5-di-tert-butyl-4-hydroxybenzyl)-2,4,6-trimethylbenzol, 1,4-Bis-(3,5-di-tert-butyl-4-hydroxybenzyl)-2,3,5,6-tetramethylbenzol, 2,4,6-Tris-(3,5-di-tert-butyl-4-hydroxybenzyl)-phenol.

1.10. Triazinverbindungen, z.B. 2,4-Bis-octylmercapto-6-(3,5-di-tert-butyl-4-hydroxyanilino)-1,3,5-triazin, 2-Octylmercapto-4,6-bis-(3,5-di-tert-butyl-4-hydroxyanilino)- 1,3,5-triazin, 2-Octylmercapto-4,6-bis-(3,5-di-tert-butyl-4-hydroxyphenoxy)-1,3,5-triazin, 2,4,6-Tris-(3,5-di-tert-butyl-4-hydroxyphenoxy)-1,2,3-triazin, 1,3,5-Tris-(3,5-di-tert-butyl-4-hydroxybenzyl)-isocyanurat, 1,3,5-Tris-(4-tert-butyl-3-hydroxy-2,6-dimethylbenzyl)-isocyanurat, 2,4,6-Tris-(3,5-di-tert-butyl-4-hydroxyphenylethyl)-1,3,5-triazin, 1,3,5-Tris-(3,5-di-tert-butyl-4-hydroxyphenylpropionyl)-hexahydro-1,3,5-triazin, 1,3,5-Tris-(3,5-dicyclohexyl-4-hydroxybenzyl)-isocyanurat.

1.11. Benzylphosphonate, z.B. Dimethyl-2,5-di-tert-butyl-4-hydroxybenzylphosphonat, Diethyl-3,5-di-tert-butyl-4-hydroxybenzylphosphonat, Dioctadecyl-3,5-di-tert-butyl-4-hydroxybenzylphosphonat, Dioctadecyl-5-tert-butyl-4-hydroxy-3-methylbenzylphosphonat, Ca-Salz des 3,5-Di-tert-butyl-4-hydroxybenzyl-phosphonsäure-monoethylesters.

1.12. Acylaminophenole, z.B. 4-Hydroxy-laurinsäureanilid, 4-Hydroxystearinsäureanilid, N-(3,5-di-tert-butyl-4-hydroxyphenyl)-carbaminsäureoctylester.

1.13. Ester der β-(3,5-Di-tert-butyl-4-hydroxyphenyl)-propionsäure mit ein- oder mehr- wertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.14. Ester der β-(5-tert-Butyl-4-hydroxy-3-methylphenyl)-propionsäure mit ein- oder mehrwertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy) ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.15. Ester der β-(3,5-Dicyclohexyl-4-hydroxyphenyl)-propionsäure mit ein- oder mehr- wertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy) ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.16. Ester der 3,5-Di-tert-butyl-4-hydroxyphenylessigsäure mit ein- oder mehrwertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.17. Amide der β-(3,5-Di-tert-butyl-4-hydroxyphenyl)-propionsäure, wie z.B. N,N'-Bis- (3,5-di-tert-butyl-4-hydroxyphenylpropionyl)-hexamethylendiamin, N,N'-Bis-(3,5-di-tert-butyl-4-hydroxyphenylpropionyl)-trimethylendiamin, N,N'-Bis-(3,5-di-tert-butyl-4-hydroxyphenylpropionyl)-hydrazin.

2. UV-Absorber und Lichtschutzmittel

2.1. 2-(2'-Hydroxyphenyl)-benzotriazole, wie z.B. 2-(2'-Hydroxy-5'-methylphenyl)-benzotriazol, 2-(3',5'-Di-tert-butyl-2'-hydroxyphenyl)-benzotriazol, 2-(5'-tert-Butyl-2'-hydroxyphenyl)-benzotriazol, 2-(2'-Hydroxy-5'-(1,1,3,3-tetramethylbutyl)phenyl)-benzotriazol, 2-(3',5'-Di-tert-butyl-2'-hydroxyphenyl)-5-chlor-benzotriazol, 2-(3'-tert-Butyl-2'-hydroxy-5'-methylphenyl)-5-chlor-benzotriazol, 2-(3'-sec-Butyl-5'-tert-butyl-2'-hydroxyphenyl)-benzotriazol, 2-(2'-Hydroxy-4'-octoxyphenyl)-benzotriazol, 2-(3',5'-Di-tert-amyl-2'-hydroxyphenyl)-benzotriazol, 2-(3',5'-Bis-(α,α-dimethylbenzyl)-2'-hydroxyphenyl)-benzotriazol, Mischung aus 2-(3'-tert-Butyl-2'-hydroxy-5'-(2-octyloxycarbonylethyl)phenyl)-5-chlor-benzotriazol, 2-(3'-tert-Butyl-5'-[2-(2-ethylhexyloxy)-carbonylethyl]-2'-hydroxyphenyl)-5-chlor-benzotriazol, 2-(3'-tert-Butyl-2'-hydroxy-5'-(2-methoxycarbonylethyl) phenyl)-5-chlor-benzotriazol, 2-(3'-tert-Butyl-2(-hydroxy-5'-(2-methoxycarbonylethyl)phenyl)-benzotriazol, 2-(3'-tert-Butyl-2'-hydroxy-5'-(2-octyloxycarbonylethyl)phenyl)-benzotriazol, 2-(3'-tert-Butyl-5'-[2-(2-ethylhexyloxy)carbonylethyl]-2'-hydroxyphenyl)-benzotriazol, 2-(3'-Dodecyl-2'-hydroxy-5'-methylphenyl)-benzotriazol, und 2-(3'-tert-Butyl-2'-hydroxy-5'-(2-isooctyloxycarbonylethyl)phenyl-benzotriazol, 2,2'-Methylen-bis[4-(1,1,3,3-tetramethylbutyl)-6-benzotriazol-2-yl-phenol]; Umesterungsprodukt von 2-[3'-tert-Butyl-5'-(2-methoxycarbonylethyl)-2'-hydroxy-phenyl]-benzotriazol mit Polyethylenglycol 300;

$$[R-CH_2CH_2-COO(CH_2)_3]_2-$$

mit R = 3'-tert-Butyl-4'-hydroxy-5'-2H-benzotriazol-2-yl-phenyl.

2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

2.3. Ester von gegebenenfalls substituierten Benzoesäuren, wie z.B. 4-tert-Butyl-phenylsalicylat, Phenylsalicylat, Octylphenyl-salicylat, Dibenzoylresorcin, Bis-(4-tert-butylbenzoyl)-resorcin, Benzoylresorcin, 3,5-Di-tert-butyl-4-hydroxybenzoesäure-2,4-di-tert-butylphenylester, 3,5-Di-tert-butyl-4-hydroxybenzoesäurehexadecylester, 3,5-Di-tert-butyl-4-hydroxybenzoesäure-octadecylester, 3,5-Di-tert-butyl-4-hydroxybenzoesäure-2-methyl-4,6-di-tert-butylphenylester.

2.4. Acrylate, wie z.B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.

2.5. Nickelverbindungen, wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert-butylbenzylphosphonsäure-monoalkylestern, wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.

2.6. Sterisch gehinderte Amine, wie z.B. Bis-(2,2,6,6-tetramethyl-piperidyl)-sebacat, Bis-(2,2,6,6-tetramethylpiperidyl)-succinat, Bis-(1,2,2,6,6-pentamethylpiperidyl)-sebacat, n-Butyl-3,5-di-tert-butyl-4-hydroxybenzylmalonsäure-bis(1,2,2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-Bis-(2,2,6,6-Tetramethyl-4-piperidyl)-hexamethylendiamin und 4-tert-Octylamino-2,6-dichlor-1,3,5-s-triazin, Tris-(2,2,6,6-tetramethyl-4-piperidyl)-nitrilotriacetat, Tetrakis-(2,2,6,6-tetramethyl-4-piperidyl)-1,2'3,4-butantetraoat, 1,1'-(1,2-Ethandiyl)-bis-(3,3,5,5-tetramethyl-piperazinon), 4-Benzoyl-2,2,6,6-tetramethylpiperidin, 4-Stearyloxy-2,2,6,6-tetramethylpiperidin, Bis-(1,2,2,6,6-pentamethylpiperidyl)-2-n-butyl-2-(2-hydroxy-3,5-di-tert-butylbenzyl)-malonat, 3-n-Octyl-7,7,9,9-tetramethyl-1,3,8-triazaspiro[4.5]decan-2,4-dion, Bis-(1-octyloxy-2,2,6,6-tetramethylpiperidyl)-sebacat, Bis-(1-octyloxy-2,2,6,6-tetramethylpiperidyl)-succinat, Kondensationsprodukt aus N,N'-Bis-(2,2,6,6-tetramethyl-4-piperidyl)-hexamethylendiamin und 4-Morpholino-2,6-dichlor-1,3,5-triazin, Kondensationsprodukt aus 2-Chlor-4,6-di-(4-n-butylamino-2,2,6,6-tetramethylpiperidyl)-1,3,5-triazin und 1,2-Bis-(3-aminopropylamino)äthan, Kondensationsprodukt aus 2-Chlor-4,6-di-(4-n-butylamino-1,2,2,6,6-pentamethylpiperidyl)-1,3,5-triazin und 1,2-Bis-(3-aminopropylamino)-äthan, 8-Acetyl-3-dodecyl-7,7,9,9-tetramethyl-1,3,8-triazaspiro[4.5]decan-2,4-dion, 3-Dodecyl-1-(2,2,6,6-tetramethyl-4-piperidyl)pyrrolidin-2,5-dion, 3-Dodecyl-1-(1,2,2,6,6-pentamethyl-4-piperidyl)-pyrrolidin-2,5-dion.

2.7. Oxalsäurediamide, wie z.B. 4,4'-Di-octyloxy-oxanilid, 2,2'-Diethoxy-oxanilid, 2,2'-Di-octyloxy-5,5'-di-tert-butyl-oxanilid, 2,2'-Di-dodecyloxy-5,5'di-tert-butyl-oxanilid, 2-Ethoxy-2'-ethyl-oxanilid, N,N'-Bis-(3-dimethylaminopropyl)-oxalamid, 2-Ethoxy-5-tert-butyl-2'-ethyloxanilid und dessen Gemisch mit 2-Ethoxy-2'-ethyl-5,4'-di-tert-butyl-oxanilid, Gemische von o- und p-Methoxy- sowie von o- und p-Ethoxy-di-substituierten Oxaniliden.

2.8. 2-(2-Hydroxyphenyl)-1,3,5-triazine, wie z.B. 2,4,6-Tris(2-hydroxy-4-octyloxyphenyl)-1,3,5-triazin, 2-(2-Hydroxy-4-octyloxyphenyl)-4,6-bis-(2,4-dimethylphenyl)-1,3,5-triazin, 2-(2,4-Dihydroxyphenyl)-4,6-bis (2,4-dimethylphenyl)-1,3,5-triazin, 2,4-Bis-(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethylphenyl)-1,3,5-triazin, 2-[2-hydroxy-4-(2-hydroxy-3-butyloxy-propyloxy)phenyl]-4,6-bis(2,4-dimethylphenyl)-1,3,5-triazin, 2-[2-hydroxy-4-(2-hydroxy-3-octyloxy-propyloxy)phenyl]-4,6-bis(2,4-dimethylphenyl)-1,3,5-triazin.

3. Metalldesaktivatoren, wie z.B. N,N'-Diphenyloxalsäurediamid, N-Salicylal-N'-salicyloylhydrazin, N,N'-Bis-(salicyloyl)-hydrazin, N,N'-Bis-(3,5-di-tert-butyl-4-hydroxyphenylpropionyl)-hydrazin, 3-Salicyloylamino-1,2,4-triazol, Bis- (benzyliden)-oxalsäuredihydrazid, Oxanilid, Isophthalsäure-dihydrazid, Sebacinsäure-bis-phenylhydrazid, N, N'-Diacetyl-adipinsäure-dihydrazid, N,N'-Bis-salicyloyl-oxalsäure-dihydrazid, N,N'-Bis-salicyloyl-thiopropionsäure-dihydrazid.

4. Phosphite und Phosphonite, wie z.B. Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert-butylphenyl)-phosphit, Diisodecylpentaerythrit-diphosphit, Bis-(2,4-di-tert-butylphenyl)-pentaerythritdiphosphit, Bis-(2,6-di-tert-butyl-4-methylphenyl)-pentaerythritdiphosphit, Bis-isodecyloxy-pentaerythritdiphosphit, Bis-(2,4-di-tert-butyl-6-methylphenyl)-pentaerythritdiphosphit, Bis-(2,4,6-tri-tert-butylphenyl)-pentaerythritdiphosphit, Tristearyl-sorbit-triphosphit, Tetrakis-(2,4-di-tert-butylphenyl)-4,4'-biphenylen-diphosphonit, 6-Isooctyloxy-2,4,8,10-tetra-tert-butyl-12H-dibenz[d,g]-1,3,2-dioxaphosphocin, 6-Fluor-2,4,8,10-tetra-tert-butyl-12-methyl-dibenz[d,g]-1,3,2-dioxaphosphocin, Bis-(2,4-di-tert-butyl-6-methylphenyl)-methylphosphit, Bis-(2,4-di-tert-butyl-6-methylphenyl)-ethylphosphit.

5. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.

6. Polyamidstabilisatoren, wie z.B. Kupfersalze in Kombination mit Jodiden und/oder Phosphorverbindungen und Salze des zweiwertigen Mangans.

7. Basische Co-Stabilisatoren, wie z.B. Melamin, Polyvinylpyrrolidon, Dicyandiamid, Tri-allylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Behenat, Mg-Stearat, Na-Ricinoleat, K-Palmitat, Antimonbrenzcatechinat oder Zinnbrenzcatechinat.

8. Nukleierungsmittel, wie z.B. 4-tert-Butylbenzoesäure, Adipinsäure, Diphenylessigsäure.

9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Ruß, Graphit.

10. Sonstige Zusätze, wie z.B. Weichmacher, Gleitmittel, Emulgatoren, Pigmente, Optische Aufheller, Flammschutzmittel, Antistatika, Treibmittel.

11. Benzofuranone bzw. Indolinone, wie z.B. in US-A-4 325 863, US-A-4 338 244, US-A-5 175 312, US-A-5 216 052, US-A-5 252 643, DE-A-4 316 611, DE-A-4 316 622, DE-A-4 316 876, EP-A-0 589 839 oder EP-A-0 591 102 beschrieben, oder 3-[4-(2-Acetoxyethoxy)phenyl]-5,7-di-tert-butyl-benzofuran-2-on, 5,7-Di-tert-butyl-3-[4-(2-stearoyloxyethoxy)phenyl]-benzofuran-2-on, 3,3'-Bis-[5,7-di-tert-butyl-3-(4-[2-hydroxyethoxy]-phenyl)-benzofuran-2-on], 5,7-Di-tert-butyl-3-(4-ethoxyphenyl)benzofuran-2-on, 3-(4-Acetoxy-3,5-dimethylphenyl)-5,7-di-tert-butyl-benzofuran-2-on, 3-(3,5-Dimethyl-4-pivaloyloxy-phenyl)-5,7-di-tert-butyl-benzofuran-2-on.

Die nachfolgenden Beispiele illustrieren die Erfindung weiter. Alle Angaben in Teilen oder Prozenten, in den Beispielen ebenso wie in der übrigen Beschreibung sowie in den Patentansprüchen, beziehen sich auf das Gewicht, sofern nichts anderes angegeben ist. In den Beispielen werden die folgenden Abkürzungen verwendet:
GC: Gaschromatographie;
HPLC Hochdruck-Flüssigchromatographie;
DSC: Differential-Thermoanalyse;
H-NMR: Magnet. Kernresonanz des Nuklids $^1$H.

A) Herstellungsbeispiele

A1) 2-Butin-1,4-diol-ditosylat (Verbindung 1) wird hergestellt gemäß Angew. Chem. 104, 1652-1654 (1992).

A2) Herstellung von 1,4-Bis-(4-Hexyloxy-2,2,6,6-tetramethylpiperidinyl)-but-2-in

A2a) Herstellung von 4-Hexyloxy-2,2,6,6-tetramethylpiperidin

In einem 2,5 Liter Sulfierkolben mit Glasrührer, Kühler und 500 ml Tropftrichter werden 314,6 g (2 Mol) 4-Hydroxy-2,2,6,6-tetramethylpiperidin, 100 g (0,1 Mol) Polyethylenglykol 1000, 1,4 l Toluol und 561 g (10 Mol) KOH pulverisiert unter Stickstoff vorgelegt.
Die Suspension wird auf 80°C erwärmt.
Während 45 Minuten werden 363,2 g (2,2 Mol) 1-Bromhexan zur gelben Lösung zugetropft.
Man läßt während 5 Std. bei 80°C rühren, wobei KBr ausfällt. Nach dem Abkühlen verdünnt man die Reaktionsmischung mit Wasser und Toluol, trennt die org. Phase ab, wäscht sie neutral und trocknet mit $Na_2SO_4$. Am Rotationsverdampfer wird das Lösungsmittel abgedampft.
Der Rückstand wird bei 76°C/0,08 Torr destilliert.
Man erhält 205 g (43 %) Titelprodukt (Verbindung 2a), das >99 % Reinheit besitzt (GC-Analyse)

| Mikroanalyse | | |
|---|---|---|
| | berechnet | gefunden |
| %C | 74,63 | 74,82 |
| %H | 12,94 | 13,04 |
| %N | 5,80 | 5,66 |

H-NMR ($CDCl_3$)

| | | | |
|---|---|---|---|
| 0,68 ppm | (1 H, s) | : | NH |
| 0,87-0,91 ppm | (3 H, t) | : | $CH_3$ (Hexyl |
| 0,96-1,04 ppm | (2 H, t) | : | $CH_2$ (Piperidin) |
| 1,14 und 1,19 ppm | (12 H, s) | : | $CH_3$ (Piperidin) |
| 1,31-1,4 ppm | (6 H, m) | : | $-(CH_2)_3-$(Hexyl) |
| 1,53-1,63 ppm | (2 H, m) | : | $-O-C-CH_2$ (Hexyl) |
| 1,93-1,98 ppm | (2 H, m) | : | $CH_2$ (Piperidin) |
| 3,44-3,49 ppm | (2 H, t) | : | $-O-CH_2-$ |
| 3,61-3,68 ppm | (1 H, m) | : | 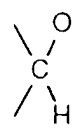 |

A2b) 1,4-Bis-(4-Hexyloxy-2,2,6,6-tetramethylpiperidinyl)-but-2-in

In einem 1 l Rundkolben mit Magnetrührer und Kühler werden unter Argon 193,1 g (0,8 Mol) der Verbindung 2a), 63,1 g (0,16 Mol) der Verbindung 1 und 500 ml Acetonitril vorgelegt.
Die Mischung wird über Nacht am Rückfluß gekocht.
Dann wird auf 700 g Eis gegossen und mit 500 ml Essigester extrahiert. Die organische Phase wird getrocknet und eingedampft. Der Rückstand wird in Hexan/Essigester = 2:1 aufgenommen und über Kieselgel filtriert. Man erhält nach dem Abtreiben des Lösungsmittels 49,5 g (58 %) des Titelproduktes (Verbindung 2) als klare, viskose Flüssigkeit.

| Mikroanalyse | | |
|---|---|---|
| | berechnet | gefunden |
| %C | 76,63 | 76,62 |
| %H | 12,11 | 12,04 |
| %N | 5,26 | 5,32 |

<u>H-NMR ($CDCl_3$)</u>

| | | |
|---|---|---|
| 0,86-0,91 ppm | (6 H, t) | : $CH_3$ (Hexyl) |
| 1,08 und 1,21 ppm | (24 H, s) | : $CH_3$ (Piperidin) |
| 1,29-1,43 ppm | (16 H, m) | : $CH_2$ (Piperidin), $-(CH_2)_3-$ (Hexyl) |
| 1,50-1,57 ppm | (4 H, m) | : $O-C-CH_2-$ (Hexyl) |
| 1,78-1,83 ppm | (4 H, m) | : $CH_2$ (Piperidin) |
| 3,32 ppm | (4 H, s) | : $N-CH_2-C{\equiv}C$ |
| 3,40-3,45 ppm | (4 H, t) | : $-O-CH_2-$ |
| 3,53-3,58 ppm | (2 H, m) | : 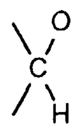 |

A3) <u>Herstellung von 1,4-Bis-(4-Benzyloxy-2,2,6,6-tetramethylpiperidinyl)-but-2-in</u>

A3a) <u>Herstellung von 4-Benzyloxy-2,2,6,6-tetramethylpiperidin</u>

Analog Beispiel A2a) werden 314,6 g (2 Mol) 4-Hydroxy-2,2,6,6-tetramethylpiperidin mit 376,2 g (2,2 Mol) Benzylbromid zur Reaktion gebracht.
Das Rohprodukt wird bei 84°C/0,006 Torr destilliert.
Man erhält 280 g (57 %) einer Flüssigkeit, die allmählich kristallisiert.
Schmelzpunkt: 30°C

| Mikroanalyse | | |
|---|---|---|
| | berechnet | gefunden |
| %C | 77,68 | 77,66 |
| %H | 10,19 | 10,29 |
| %N | 5,66 | 5,60 |

<u>H-NMR ($CDCl_3$)</u>

| | | | |
|---|---|---|---|
| 0,71 ppm | (1 H, s) | : | NH |
| 1,05 und 1,13 ppm | (2 H, t) | : | $CH_2$ (Piperidin) |
| 1,15-1,17 ppm | (12 H, s) | : | $CH_3$ |
| 1,99-2,05 ppm | (2 H, m) | : | $CH_2$ (Piperidin) |
| 3,76-3,86 ppm | (1 H, m) | : | 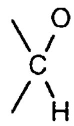 |
| 4,58 ppm | (2 H, s) | : | $-O-CH_2-$ |
| 7,26-7,35 ppm | (5 H, m) | : | Aromat |

A3b) <u>1,4-Bis-(4-Benzyloxy-2,2,6,6-tetramethylpiperidin)-but-2-in</u>

Analog Beispiel A2b) werden 202 g (0,84 Mol) der Verbindung aus Beispiel A3a) mit 41,4 g (0,17 Mol) 2-Butin-1,4-diol-ditosylat (Verbindung 1) in 500 ml Acetonitril zur Reaktion gebracht.
Das Rohprodukt wird in Methanol umkristallisiert.
Man erhält 38,2 g (41 %) des Titelproduktes als Feststoff, der bei 80,5°C schmilzt.

| Mikroanalyse | | |
|---|---|---|
| | berechnet | gefunden |
| %C | 79,36 | 79,35 |
| %H | 9,62 | 9,81 |
| %N | 5,14 | 5,06 |

<u>H-NMR ($CDCl_3$)</u>

| | | | |
|---|---|---|---|
| 1,06 und 1,23 ppm | (24 H, s) | : | $CH_3$ |
| 1,42-1,50 ppm | (4 H, t) | : | $CH_2$ (Piperidin) |
| 1,84-1,89 ppm | (4 H, m) | : | $CH_2$ (Piperidin) |
| 3,33 ppm | (4 H, s) | : | $N-CH_2-C\equiv C$ |
| 3,64-3,74 ppm | (2 H, m) | : | 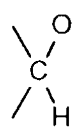 |
| 4,54 ppm | (4 H, s) | : | $-O-CH_2-$ |
| 7,24-7,42 ppm | (10 H, m) | : | Aromat |

A4) <u>Herstellung von 1,4-Bis-(4-Methoxy-2,2,6,6-tetramethylpiperidinyl)-but-2-in</u>

A4a) <u>Herstellung von 4-Methoxy-2,2,6,6-tetramethylpiperidin</u>

In einen 2,5 l Sulfierkolben mit Glasrührer, Thermometer und Kühler werden 236 g (1,5 Mol) 4-Hydroxy-2,2,6,6-tetramethylpiperidin, 4 g (15 mmol) 18-Crown-6, 420 g (7,5 Mol) KOH pulverisiert und 30 g (0,21 Mol) Natriumsulfat zu 1,5 1 THF gegeben. Die Mischung wird auf 35°C erwärmt.

In 4 Std. werden 212 g (1,5 Mol) Methyljodid, gelöst in 150 ml THF, zugetropft.
Man gießt dann auf Eis/Essigester und trennt die organische Phase ab. Diese wird getrocknet und vorsichtig am Rotationsverdampfer eingedampft.
Der Rückstand wird bei 67°C/16 mm destilliert.
Man erhält 190 g (74 %) einer klaren Flüssigkeit. GC-Reinheit: 95 %

| Mikroanalyse | | |
|---|---|---|
| | berechnet | gefunden |
| %C | 70,12 | 70,31 |
| %H | 12,36 | 12,39 |
| %N | 8,18 | 8,04 |

H-NMR ($CDCl_3$)

| | | |
|---|---|---|
| 0,71 ppm | (1 H, s) | : NH |
| 0,97-1,03 ppm | (2 H, t) | : $CH_2$ |
| 1,15 und 1,19 ppm | (12 H, s) | : $CH_3$ (Piperidin) |
| 1,95-2,00 ppm | (2 H, m) | : $CH_2$ |
| 3,37 ppm | (3 H, s) | : O-$CH_3$ |
| 3,52-3,62 ppm | (1 H, m) | : 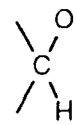 |

A4b) 1,4-Bis-(4-Methoxy-2,2,6,6-tetramethylpiperidinyl)-but-2-in

Analog Beispiel A2b) werden 188,4 g (1,1 Mol) der Verbindung aus Beispiel A4a) mit 53,1 g (0,22 Mol) 2-Butin-1,4-diol-ditosylat (Verbindung 1) im 650 ml Acetonitril zur Reaktion gebracht.
Das Rohprodukt wird bei 170°C/0,08 Torr destilliert und anschließend kristallisiert;
Schmelzpunkt 62°C, Ausbeute 40 g (46 %).

| Mikroanalyse | berechnet | gefunden |
|---|---|---|
| %C | 73,42 | 73,17 |
| %H | 11,30 | 11,43 |
| %N | 7,13 | 6,91 |

H-NMR ($CDCl_3$)

| | | |
|---|---|---|
| 1,08 und 1,23 ppm | (24 H, s) | : $CH_3$ (Piperidin) |
| 1,29-1,37 ppm | (4 H, t) | : $CH_2$ (Piperidin) |
| 1,80-1,86 ppm | (4 H, m) | : $CH_2$ (Piperidin) |
| 3,33 ppm | (10 H, s) | : N-$CH_2$-C≡C und -O-$CH_3$ |
| 3,42-3,52 ppm | (2 H, m) | : 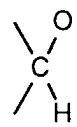 |

A5) Herstellung von 1,4-Bis-(4-Hydroxy-2,2,6,6-tetramethylpiperidinyl)-but-2-in

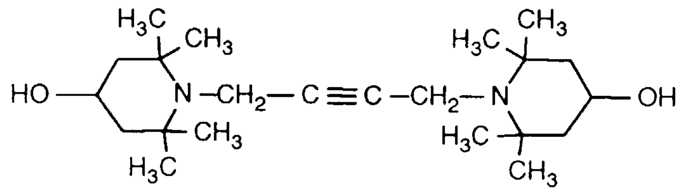

Analog Beispiel A2b) werden 833 g (5,3 Mol) 4-Hydroxy-2,2,6,6-tetramethylpiperidin und 418 g (1,05 Mol) 2-Butin-1,4-diol-ditosylat (Verbindung 1) in 3,61 Acetonitril zur Reaktion gebracht.
Das Rohprodukt wird aus Ethanol umkristallisiert. Man erhält 295 g (76 %) des Titelproduktes als weiße Kristalle mit einem Schmelzpunkt von 203,4°C.

| Mikroanalyse | berechnet | gefunden |
|---|---|---|
| %C | 72,48 | 72,52 |
| %H | 11,06 | 11,12 |
| %N | 7,68 | 7,75 |

H-NMR (DMSO)

| | | | |
|---|---|---|---|
| 1,01 und 1,11 ppm | (24 H, s) | : | $CH_3$ |
| 1,11-1,19 ppm | (4 H, t) | : | $CH_2$ (Piperidin) |
| 1,60-1,66 ppm | (4 H, m) | : | $CH_2$ (Piperidin) |
| 3,34 ppm | (4 H, s) | : | $N-CH_2-C\equiv C$ |
| 3,67-3,76 ppm | (2 H, m) | : | 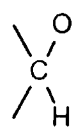 |
| 4,37-4,39 ppm | (2 H, d) | : | -OH |

A6) Herstellung von 1,4-Bis-(4-[n-Octylcarbonyloxy]-2,2,6,6-tetramethylpiperidinyl)-but-2-in

In einem 21 Rundkolben mit Magnetrührer werden 72,9 g (0,2 Mol) 1,4-Bis-(4-Hydroxy-2,2,6,6-tetramethylpiperidinyl)-but-2-in (Verbindung 5), 86,2 g (0,5 Mol) Pelargonsäuremethylester, 8 g Dibutylzinnoxid und 800 ml Xylol vorgelegt. Dem Rundkolben wird eine Vigreux-Destillationseinheit aufgesetzt. Man erwärmt auf 150°C und läßt zuerst das Methanol abdestillieren. Dann wird die Temperatur noch etwas erhöht, so daß auch Xylol langsam destilliert. Der Rückstand destilliert man bei 60°C/0,008 Torr.
Man erhält 99 g (77 %) viskose Flüssigkeit.

| Mikroanalyse | | |
|---|---|---|
| | berechnet | gefunden |
| %C | 74,48 | 74,25 |
| %H | 11,25 | 11,23 |
| %N | 4,34 | 4,24 |

<u>H-NMR ($CDCl_3$)</u>

| | | | |
|---|---|---|---|
| 0,86-0,9 ppm | (6 H, t) | : | $CH_3$ (Pelargonsäure) |
| 1,13 und 1,22 ppm | (24 H, s) | : | $CH_3$ (Piperidin) |
| 1,20-1,4 ppm | (20 H, m) | : | $-(CH_2)_5-$ (Pelargonsäure) |
| 1,45-1,57 ppm | (4 H, t) | : | $CH_2$ (Piperidin) |
| 1,56-1,62 ppm | (4 H, m) | : | $OCC-C-CH_2-$ |
| 1,76-1,82 ppm | (4 H, m) | : | $CH_2$ (Piperidin) |
| 2,23-2,32 ppm | (4 H, t) | : | $OOC-CH_2-$ |
| 3,32 ppm | (4 H, s) | : | $N-CH_2-C{\equiv}C$ |
| 5,03-5,11 ppm | (2 H, m) | : | 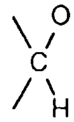 |

A7) <u>Herstellung von 1,4-Bis-(4-[n-Heptadecyl-carbonyloxy]-2,2,6,6-tetramethylpiperidinyl)-but-2-in</u>

Analog Beispiel A6) werden 48,1 g (132 mmol) der Verbindung 5 und 100 g (0,33 Mol) Stearinsäuremethylester zur Reaktion gebracht.
Das Rohprodukt wird in Ethanol umkristallisiert.
Man erhält 88,6 g (75 %) weiße Kristalle mit einem Schmelzpunkt von 68°C.

| Mikroanalyse | | |
|---|---|---|
| | berechnet | gefunden |
| %C | 77,62 | 77,53 |
| %H | 12,13 | 12,19 |
| %N | 3,12 | 3,14 |

<u>H-NMR ($CDCl_3$)</u>

| | | | |
|---|---|---|---|
| 0,86-0,9 ppm | (6 H, t) | : | $CH_3$ (Stearinsäure) |
| 1,13 und 1,22 ppm | (24 H, s) | : | $CH_3$ (Piperidin) |
| 1,20-1,35 ppm | (56 H, m) | : | $-(CH_2)_{14}-$ |
| 1,46-1,49 ppm | (4 H, t) | : | $CH_2$ (Piperidin) |
| 1,48-1,62 ppm | (4 H, m) | : | $OOC-C-CH_2-$ |
| 1,76-1,81 ppm | (4 H, m) | : | $CH_2$ (Piperidin) |
| 2,23-2,28 ppm | (4 H, t) | : | $OOC-CH_2-$ |
| 3,32 ppm | (4 H, s) | : | $N-CH_2-C{\equiv}C$ |
| 5,02-5,10 ppm | (2 H, m) | : | 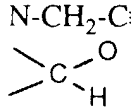 |

A8) <u>Herstellung von 1,4-Bis-(4-[n-Pentadecylcarbonyloxy]-2,2,6,6-tetramethylpiperidinyl)-but-2-in</u>

Analog Beispiel A6) werden 72,9 g (0,2 Mol) 1,4-Bis-(4-Hydroxy-2,2,6,6-tetramethyl-piperidinyl)-but-2-in und

135,2 g (0,5 Mol) Palmitinsäuremethylester zur Reaktion gebracht.
Das Rohprodukt wird in Ethanol umkristallisiert. Man erhält 121 g (72 %) weiße Kristalle mit einem Schmelzpunkt von 61°C.

| Mikroanalyse | | |
|---|---|---|
| | berechnet | gefunden |
| %C | 77,08 | 76,93 |
| %H | 11,98 | 11,99 |
| %N | 3,33 | 3,32 |

<u>H-NMR ($CDCl_3$)</u>

| | | | |
|---|---|---|---|
| 0,86-0,9 ppm | (6 H, t) | : | $CH_3$ (Palmitinsäure) |
| 1,12 und 1,22 ppm | (24 H, s) | : | $CH_3$ (Piperidin) |
| 1,20-1,40 ppm | (48 H, m) | : | $-(CH_2)_{12}-$ |
| 1,45-1,54 ppm | (4 H, t) | : | $CH_2$ (Piperidin) |
| 1,53-1,68 ppm | (4 H, m) | : | $OOC-C-CH_2-$ |
| 1,76-1,82 ppm | (4 H, m) | : | $CH_2$ (Piperidin) |
| 2,23-2,30 ppm | (4 H, t) | : | $OOC-CH_2-$ |
| 3,32 ppm | (4 H, s) | : | $N-CH_2-C{\equiv}C$ |
| 5,02-5,10 ppm | (2 H, m) | : | 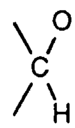 |

A9) <u>Herstellung von 1,4-Bis-(4-[Ethylcarbonyloxy]-2,2,6,6-tetramethylpiperidinyl)-but-2-in</u>

In einem 1,5 l Sulfierkolben mit Glasrührer, Thermometer, Kühler und Tropftrichter werden 100 g (274 mmol) der Verbindung aus Beispiel A5), 83,1 g (823 mmol) Triethylamin und 1 l THF vorgelegt. Bei 20°C werden 76,1 g (823 mmol) Propionsäurechlorid, gelöst in 100 ml THF, zugetropft. Man läßt über Nacht bei 25°C rühren.
Man trennt von unlöslichem Salz ab und dampft dann das Lösungsmittel ab.
Der Rückstand wird in Methanol umkristallisiert.
Man erhält 52 g (39 %) einer Substanz, die bei 78°C schmilzt.

| Mikroanalyse | | |
|---|---|---|
| | berechnet | gefunden |
| %C | 70,55 | 70,44 |
| %H | 10,15 | 10,12 |
| %N | 5,88 | 5,68 |

H-NMR ($CDCl_3$)

| | | | |
|---|---|---|---|
| 1,09-1,15 ppm | (18 H, s,t) | : | $CH_3$ (Propionsäure), $CH_3$ (Piperidin) |
| 1,22 ppm | (12 H, s) | : | $CH_3$ (Piperidin) |
| 1,43-1,53 ppm | (4 H, t) | : | $CH_2$ (Piperidin) |
| 1,77-1,82 ppm | (4 H, m) | : | $CH_2$ (Piperidin) |
| 2,25-2,33 ppm | (4 H, q) | : | $OOC-CH_2-$ |
| 3,32 ppm | (4 H, s) | : | $N-CH_2-C\equiv C$ |
| 5,02-5,12 ppm | (2 H, m) | : | 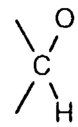 |

A10) Herstellung von 1,4-Bis-(4-Acetoxy-2,2,6,6-tetramethylpiperidinyl)-but-2-in

A10a) Herstellung von 4-Acetoxy-2,2,6,6-tetramethylpiperidin

In einem 1,5 l Sulfierkolben mit Glasrührer, Thermometer und Kühler werden 157 g (1 Mol) 4-Hydroxy-2,2,6,6-tetramethylpiperidin, 60 g (1 Mol) Essigsäure, 300 g (3 Mol) Essigsäureanhydrid und 5 Tropfen konzentrierte Schwefelsäure eingebracht. Unter Stickstoff läßt man über Nacht bei 60 °C rühren. Dann läßt man auf 25°C abkühlen und gießt eiskalte Natronlauge bis pH 10 dazu.
Dann wird mit Diethylether extrahiert, die org. Phase getrocknet, filtriert und eingedampft.
Der Rückstand wird bei 102°C/19 Torr destilliert. Man erhält 150 g (75 %) einer klaren Flüssigkeit. GC-Reinheit ca. 98 %.

| Mikroanalyse | | |
|---|---|---|
| | berechnet | gefunden |
| %C | 66,29 | 66,03 |
| %H | 10,62 | 10,76 |
| %N | 7,03 | 6,93 |

H-NMR ($CDCl_3$)

| | | | |
|---|---|---|---|
| 0,97 ppm | (1 H, s) | : | NH |
| 1,07-1,18 ppm | (8 H, s,t) | : | $CH_3$ und $CH_2$ (Piperidin) |
| 1,23 ppm | (6 H, s) | : | $CH_3$ (Piperidin) |
| 1,89-1,98 ppm | (2 H, m) | : | $CH_2$ |
| 2,11 ppm | (3 H, s) | : | $OOC-CH_3$ |
| 5,13-5,23 ppm | (1 H, m) | : | 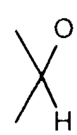 |

A10b) 1,4-Bis-(4-Acetoxy-2,2,6,6-tetramethylpiperidinyl)-but-2-in

Analog Beispiel A2b) werden 39,5 g (0,1 Mol) 2-Butin-1,4-diol-ditosylat und 59,8 g (0,3 Mol) 4-Acetoxy-2,2,6,6-tetramethylpiperidin (Verbindung 10a) in 360 ml Acetonitril zur Reaktion gebracht.
Das Rohprodukt wird in Acetonitril umkristallisiert.
Man erhält 20,3 g (45 %) eines farblosen Feststoffs, der bei 113°C schmilzt.

| Mikroanalyse | | |
|---|---|---|
| | berechnet | gefunden |
| %C | 69,61 | 69,65 |
| %H | 9,89 | 9,83 |
| %N | 6,24 | 6,25 |

H-NMR ($CDCl_3$)

| | | | |
|---|---|---|---|
| 1,12 und 1,23 ppm | (24 H, s) | : | $CH_3$ (Piperidin) |
| 1,43-1,53 ppm | (4 H, t) | : | $CH_2$ (Piperidin) |
| 1,77-1,82 ppm | (4 H, m) | : | $CH_2$ (Piperidin) |
| 2,02 ppm | (6 H, s) | : | $OOC-CH_3$ |
| 3,32 ppm | (4 H, s) | : | $N-CH_2-C\equiv C$ |
| 5,00-5,11 ppm | (2 H, m) | : | 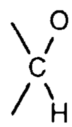 |

A11) Herstellung von 1,4-Bis-(4-Acetamido-2,2,6,6-tetramethylpiperidinyl)-but-2-in

A11a) Herstellung von 4-Acetamido-2,2,6,6-tetramethylpiperidin

In einem 10 1 Reaktor mit Glasrührer, Thermometer und Tropftrichter werden 625 g (4 Mol) 4-Amino-2,2,6,6-tetramethylpiperidin in 2,8 l Toluol gelöst. Dazu tropft man eine Lösung aus 450 g (4,4 Mol) Essigsäureanhydrid in 400 ml Toluol, so daß die Temperatur unterhalb 25°C bleibt. Nach der Zugabe läßt man noch etwa 1 Stunde rühren. Man gießt dann 4 l 2n Natronlauge zur Reaktionslösung, mischt gut durch und trennt die org. Phase ab. Die organische Phase wird getrocknet, eingedampft und der Rückstand in Acetonitril umkristallisiert.
Man erhält 687 g (80 %) des Titelproduktes in einer kristallinen, Kristallwasser-haltigen Modifikation vom Schmelzpunkt 125°C.

| Mikroanalyse | | |
|---|---|---|
| | berechnet | gefunden |
| %C | 61,06 | 61,09 |
| %H | 11,18 | 11,30 |
| %N | 12,98 | 13,00 |

H-NMR ($CD_3OD$)

| | | | |
|---|---|---|---|
| 1,00-1,08 ppm | (2 H, t) | : | $CH_2$ |
| 1,13 und 1,23 ppm | (12 H, s) | : | $CH_3$ (Piperidin) |
| 1,73-1,78 ppm | (2 H, m) | : | $CH_2$ |
| 1,91 ppm | (3 H, s) | : | $OC\text{-}CH_3$ |
| 4,11-4,18 ppm | (1 H, m) | : | 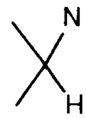 |

A11b) 1,4-Bis-(4-Acetamido-2,2,6,6-tetramethylpiperidinyl)-but-2-in

Analog Beispiel A2b) werden 79 g (0,2 Mol) 2-Butin-1,4-diol-ditosylat und 159 g (0,8 Mol) 4-Acetamid-2,2,6,6-tetramethylpiperidin aus Beispiel A11a) in 700 ml Acetonitril zur Reaktion gebracht.
Das Rohprodukt wird in Dioxan umkristallisiert. Man erhält 42 g (47 %) des Titelproduktes als farblose Substanz, die sich bei 251°C zersetzt.

| Mikroanalyse: | berechnet | gefunden |
|---|---|---|
| %C | 69,91 | 69,70 |
| %H | 10,38 | 10,50 |
| %N | 12,54 | 12,47 |

H-NMR ($CD_3OD$)

| | | | |
|---|---|---|---|
| 1,15 und 1.21 ppm | (24 H, s) | : | $CH_3$ (Piperidin) |
| 1,23-1.35 ppm | (4 H, t) | : | $CH_2$ (Piperidin) |
| 1,62-1,67 ppm | (4 H, m) | : | $CH_2$ (Piperidin) |
| 1,91 ppm | (6 H, s) | : | $OC\text{-}CH_3$ |
| 3,37 ppm | (4 H, s) | : | $N\text{-}CH_2\text{-}C{\equiv}C$ |
| 4,04-4,13 ppm | (2 H, m) | : | 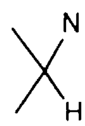 |

A12) 1,4-Bis-(4-Allyloxy-2,2,6,6-tetramethylpiperidinyl)-but-2-in

A12a) 4-Allyloxy-2,2,6,6-tetramethylpiperidin

Analog Beispiel A2a) werden 156 g (1 Mol) 4-Hydroxy-2,2,6,6-tetramethylpiperidin mit 145 g (1,2 Mol) Allylbromid zur Reaktion gebracht.
Man erhält nach der Destillation des Rohproduktes 159 g (81 %) einer Flüssigkeit mit Siedepunkt 40°C/3 mbar. Die Reinheit ist > 98 %. (GC)

| Mikroanalyse: | berechnet | gefunden |
|---|---|---|
| %C | 73,04 | 72,94 |
| %H | 11,75 | 11,73 |
| %N | 7,10 | 7,08 |

A12b) 1,4-Bis-(4-Allyloxy-2,2,6,6-tetramethylpiperidinyl)-but-2-in

Analog Beispiel A2b) werden 148 g (750 mmol) 4-Allyloxy-2,2,6,6-tetramethylpiperidin und 59 g (150 mmol) 2-Butin-1,4-diol-ditosylat in 500 ml Acetonitril zur Reaktion gebracht.
Das Rohprodukt wird bei 170°C/0,08 Torr destilliert. Man erhält 50,2 g (75 %) des Titelproduktes (Verbindung 12) vom Schmelzpunkt 53°C.

| Mikroanalyse: | berechnet | gefunden |
|---|---|---|
| %C | 75,63 | 75,53 |
| %H | 10,88 | 11,08 |
| %N | 6,30 | 6,34 |

H-NMR ($CDCl_3$)

| | | | |
|---|---|---|---|
| 1,07 und 1,22 ppm | (24 H, s) | : | $CH_3$ |
| 1,28-1,43 ppm | (4 H, t) | : | $CH_2$ (Piperidin) |
| 1,79-1,84 ppm | (4 H, m) | : | $CH_2$ (Piperidin) |
| 3,32 ppm | (4 H, s) | : | $N-CH_2-C\equiv C$ |
| 3,57-3,67 ppm | (2 H, m) | : | 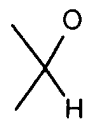 |
| 3,99-4,02 ppm | (4 H, d) | : | $-O-CH_2-$ |
| 5,12-5,30 ppm | (4H, q) | : | $=CH_2$ |
| 5,86-5,99 ppm | (2 H, m) | : | $-CH=$ |

A13) 1,4-Bis(triacetonamin-ethylenketal)-but-2-in

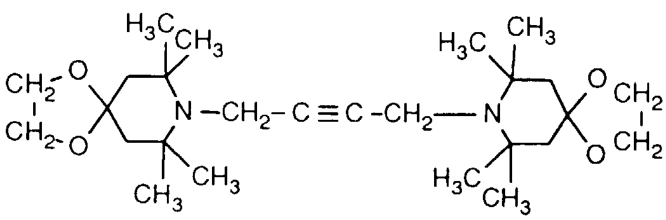

199 g (1 Mol) Triacetonamin-ethylenketal und 79 g (0,2 Mol) der Verbindung 1 werden in 700 ml Acetonitril während 5 Stunden auf Rückflußtemperatur gehalten. Das nach Abkühlen erhaltene Rohprodukt wird aus Methanol umkristallisiert. Man erhält 80 g (89 %) des Titelproduktes vom Schmelzpunkt 123°C.

| Mikroanalyse | | |
|---|---|---|
| | berechnet | gefunden |
| %C | 69,61 | 69,84 |
| %H | 9,89 | 10,04 |
| %N | 6,24 | 6,34 |

| H-NMR ($CDCl_3$) | | |
|---|---|---|
| 1,20 ppm | (24 H, s) | $CH_3$ |
| 1,68 ppm | (8 H, s) | $CH_2$ (Piperidin) |
| 3,37 ppm | (4 H, s) | N-$CH_2$- |
| 3,91 ppm | (8 H, s) | -O-$CH_2$-$CH_2$-O- |

A14) 1,4-Bis(7-dodecyl-2,2,10,10-tetramethyl-1,5,7-triazaspiro[4,5] decan-6,8-dion-1-yl)-but-2-in

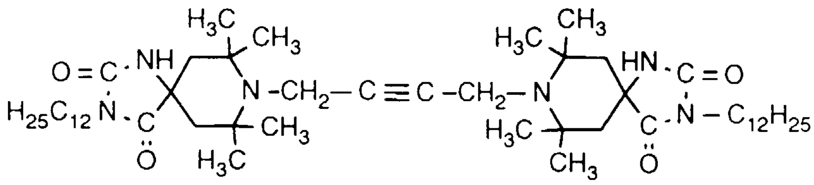

Analog Beispiel A2b) werden 39,4 g (0,1 Mol) 3-Dodecyl-7,7,9,9-tetramethyl-1,3,8-triazaspiro[4,5]decan-2,4-dion und 7,9 g (20 mMol) 2-Butin-1,4-diol-ditosylat in 420 ml Acetonitril zur Reaktion gebracht. Das Rohprodukt wird zunächst aus 300 ml n-Hexan und anschließend bis zum Erreichen des konstanten Schmelzpunktes 162°C aus Ethanol umkristallisiert.

| Mikroanalyse: | | |
|---|---|---|
| | berechnet | gefunden |
| %C | 71,73 | 71,81 |
| %H | 10,59 | 10,62 |
| %N | 10,04 | 10,09 |

A15) 2,4-Hexadiin-1,6-diol-ditosylat

In einem 1,5 l Sulfierkolben mit Thermometer, Magnetrührer, Tropftrichter und Kühler werden 54,6 g (0,5 Mol) 2,4-Hexadiin-1,6-diol, 220 g (1,16 Mol) Toluol-4-sulfonsäurechlorid und 750 ml Acetonitril vorgelegt. Unter Kühlen mit dem Eisbad werden 56 g (1 Mol) KOH als Lösung in 95 ml Wasser innerhalb von 30 Minuten zugetropft. Man läßt bei 20-25°C für 24 h rühren. Anschließend wird die Reaktionsmischung mit 700 ml Wasser und 1 l Diethylether versetzt. Die organisch Phase wird abgetrennt, mit Natriumbicarbonatlösung und Wasser gewaschen, mit Natriumsulfat getrocknet und eingedampft. Nach zweimaligem Umkristallisieren aus Methanol erhält man 32 g des Titelproduktes vom Schmelzpunkt 95°C (Verbindung 15).

| Mikroanalyse: | | |
|---|---|---|
| | berechnet | gefunden |
| %C | 57,40 | 57,38 |
| %H | 4,34 | 4,36 |
| %N | 15,32 | 15,03 |

A16) 1,6-Bis-(4-Hydroxy-2,2,6,6-tetramethylpiperidin-1-yl)-2,4-hexadiin

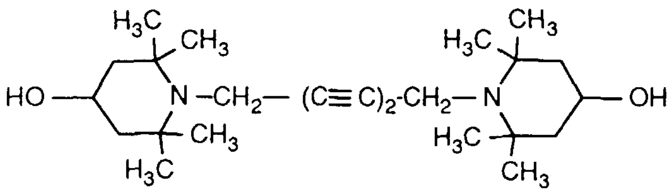

Analog Beispiel A2b) werden 2,5 g (6 mMol) der Verbindung 15 mit 4,7 g (30 mM 4-Hydroxy-2,2,6,6-tetramethylpiperidin in 30 ml Acetonitril zur Reaktion gebracht.
Das Rohprodukt wird aus Toluol umkristallisiert. Man erhält 2 g (83 %) des Titelproduktes als weiße Kristalle; Zersetzungspunkt 234°C.

| Mikroanalyse | berechnet | gefunden |
|---|---|---|
| %C | 74,18 | 74,28 |
| %H | 10,38 | 10,38 |
| %N | 7,21 | 7,16 |

A17) 1,6-Bis-(4-Benzyloxy-2,2,6,6-tetramethylpiperidin)-2,4-hexadiin

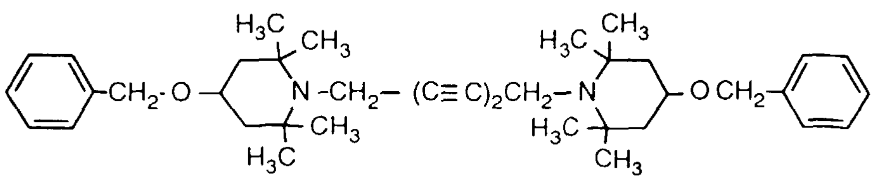

Analog Beispiel A2b) werden 15 g (36 mMol) der Verbindung 15 mit 44,5 g (180 mMol) der Verbindung aus Beispiel A3a) in 150 ml Acetonitril umgesetzt.
Das Rohprodukt wird zu gleichen Teilen jeweils aus Toluol, Acetonitril oder Essige umkristallisiert; das Endprodukt der obigen Formel ist jeweils vom Schmelzpunkt 171°C.

| Mikroanalyse: | | |
|---|---|---|
| | berechnet | gefunden |
| %C | 80,24 | 80,13 |
| %H | 9,21 | 9,31 |
| %N | 4,92 | 4,92 |

B) Anwendungsbeispiele

B1) Lichtstabilisierung von Polypropylen-Platten

Je 1 g der in obigen Beispielen beschriebenen erfindungsgemäßen Stabilisatoren werden mit 1000 g Polypropylen vom Schmelzindex 4,0 g/10 Min. (230°C; 2.16 kg) des Herstellers Statoil, Stathelle, Norwegen, und den folgenden Zusätzen gemischt:
0,5 g Pentaerythrityl-tetrakis(3-[3',5'-di-tert.butyl-4'-hydroxyphenyl]-propionat);
1,0 g Tris(2,4-di-tert.butyl-phenyl)-phosphit;

1,0 g FILOFIN® Blue G (Pigment; Ciba Geigy S.p.A., Origgio, Italien);
1,0 g Calciumstearat.

Zu Vergleichszwecken wird eine Mischung ohne erfindungsgemäßen Stabilisator hergestellt.

Die Mischung wird bei 200-230°C extrudiert. Das erhaltene Granulat wird im Spritzgußverfähren bei 200-220°C zu Platten von 2 mm Dicke verarbeitet.

Die Platten werden gemäß ASTM D 2565-85 in einem Weather-O-Meter® 65 WR (Atlas Electric Devices, Chicago, USA) bei einer Schwarztafeltemperatur von 63 ± 3 °C belichtet. Die Proben werden regelmäßig untersucht; der Beginn einer oberflächlichen Versprödung (Chalking) wird ermittelt. Die Belichtungszeit bis zum Auftreten der Versprödung ist der nachfolgenden Tabelle 1 zu entnehmen.

Tab. 1:

| Belichtungsdauer bis Oberflächenversprödung | |
|---|---|
| Stabilisator aus Beispiel | Belichtungsdauer (h) |
| ohne | 620 |
| A2 | 3420 |
| A3 | 3800 |
| A4 | 4130 |
| A12 | 3800 |

Die erfindungsgemäß stabilisierten Proben weisen eine hervorragende Lichtbeständigkeit auf.

## Patentansprüche

1. Verbindung der Formel I

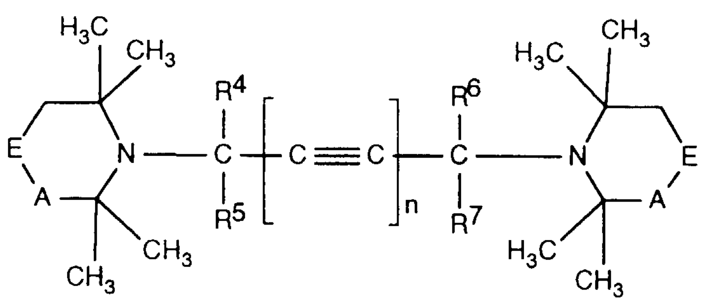

(I)

worin n 1 oder 2 ist, A $-CH_2-$ oder -CO- bedeutet;
wenn A Methylen ist, E die Bedeutung

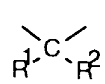

hat, und
wenn A Carbonyl ist, E die Bedeutung $-N-R^3$ hat;
$R^1$ Wasserstoff ist;
$R^2$ -OH; $-O-R^8$; $-S-R^8$; $-OCO-R^8$; $-NHCO-R^8$ oder $-N(R^{13})R^{14}$ ist; oder
$R^1$ und $R^2$ gemeinsam einen Substituenten =O bedeuten oder zusammen mit dem Kohlenstoffatom, an das sie gebunden sind, einen Fünf- oder Sechsring einer der Formeln

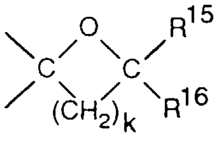, 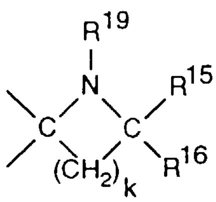, 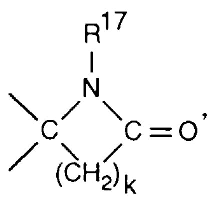, 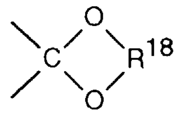, 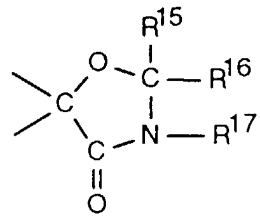,

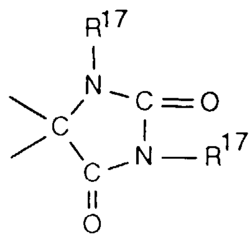

darstellen, wobei k 2 oder 3 ist;
$R^3$ $C_1$-$C_{18}$-Alkyl; $C_3$-$C_{18}$-Alkenyl; $C_5$-$C_8$-Cycloalkyl; Phenyl; Naphthyl; $C_7$-$C_9$-Phenylalkyl; oder $C_{11}$-$C_{14}$-Naphthylalkyl bedeutet;
$R^4$, $R^5$, $R^6$ und $R^7$, unabhängig voneinander, Wasserstoff oder Methyl darstellen;
$R^8$ $C_1$-$C_{50}$-Alkyl, $C_2$-$C_{18}$-Alkenyl oder Glycidyl bedeutet; oder $C_2$-$C_{50}$-Alkyl bedeutet, welches durch -O-, -S-, -$NR^{19}$- und/oder $C_5$-$C_8$-Cycloalkylen unterbrochen ist; oder $R^8$ $C_5$-$C_{12}$-Cycloalkyl, welches unsubstituiert oder durch 1 bis 4 -$R^{12}$ substituiert ist;
$C_6$-$C_{10}$-Aryl, welches unsubstituiert oder durch 1 bis 4 -$R^{12}$ oder -$OR^{12}$ substituiert ist;
oder $C_7$-$C_{50}$-Aralkyl, welches unsubstituiert oder durch $C_5$-$C_8$-Cycloalkyl substituiert und/oder im aliphatischen Teil durch $C_5$-$C_8$-Cycloalkylen unterbrochen und/oder im aliphatischen Teil durch Sauerstoff oder Schwefel unterbrochen und/oder im aromatischen Teil durch 1 bis 4 -$R^{12}$ oder -$OR^{12}$ substituiert ist, bedeutet;
$R^{12}$ $C_1$-$C_{18}$-Alkyl; $C_2$-$C_{18}$-Alkenyl; $C_5$-$C_8$-Cycloalkyl; Phenyl oder Benzyl ist;
$R^{13}$ und $R^{14}$, unabhängig voneinander, jeweils eine der für $R^8$ angegebenen Bedeutungen haben; oder
$R^{13}$ und $R^{14}$ zusammen mit dem Stickstoffatom, an das sie gebunden sind, zyklisches Imid der Formel

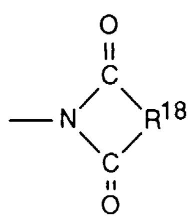

darstellen, dessen Ringstruktur 4 bis 6 Kohlenstoffatome enthält;
$R^{15}$ und $R^{16}$, unabhängig voneinander, H; oder $C_1$-$C_{12}$-Alkyl; oder gemeinsam geradkettiges, $\alpha,\omega$-verknüpftes $C_4$-$C_{13}$-Alkylen bedeuten;
$R^{17}$ H ist oder eine der Bedeutungen von $R^3$ hat;
$R^{18}$ $C_2$-$C_{18}$-Alkylen oder $C_2$-$C_6$-Alkenylen darstellt; und
$R^{19}$ eine der Bedeutungen von $R^3$ hat.

2. Verbindung der Formel I gemäß Anspruch 1, worin A Methylen ist, E die Bedeutung

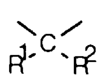

hat, und
$R^2$ -OH; -O-$R^8$; -OCO-$R^8$; -NHCO-$R^8$ oder -N($R^{13}$)$R^{14}$ ist; oder

$R^1$ und $R^2$ gemeinsam einen Substituenten =O bedeuten oder zusammen mit dem Kohlenstoffatom, an das sie gebunden sind, einen Fünf- oder Sechsring einer der Formeln

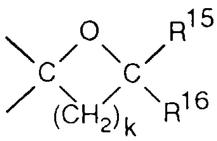, 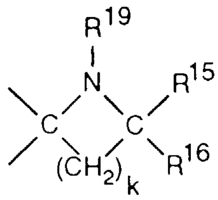, 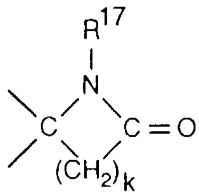, 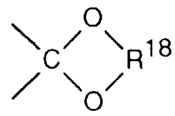, 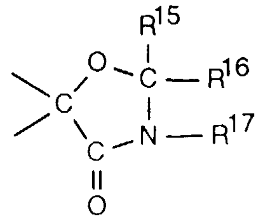,

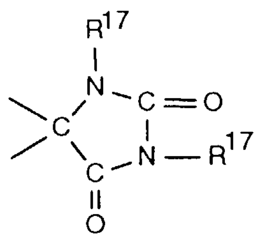

darstellen, wobei k 2 oder 3 ist;
$R^4$, $R^5$, $R^6$ und $R^7$ Wasserstoff sind,
$R^8$ $C_1$-$C_{50}$-Alkyl, $C_2$-$C_{18}$-Alkenyl oder Glycidyl bedeutet; oder $C_2$-$C_{50}$-Alkyl bedeutet, welches durch -O-, -S-, -$NR^{19}$- und/oder $C_5$-$C_8$-Cycloalkylen unterbrochen ist; oder $R^8$ $C_5$-$C_{12}$-Cycloalkyl, welches unsubstituiert oder durch 1 bis 4 -$R^{12}$ substituiert ist; Phenyl, welches unsubstituiert oder durch 1 bis 4 -$R^{12}$ oder -$OR^{12}$ substituiert ist; oder $C_7$-$C_{18}$-Aralkyl, welches unsubstituiert oder im aromatischen Teil durch 1 bis 4 -$R^{12}$ oder -$OR^{12}$ substituiert ist, bedeutet;
$R^{12}$ $C_1$-$C_{18}$-Alkyl; $C_2$-$C_{18}$-Alkenyl; $C_5$-$C_8$-Cycloalkyl; Phenyl oder Benzyl ist;
$R^{13}$ und $R^{14}$, unabhängig voneinander, jeweils eine der für $R^8$ angegebenen Bedeutungen haben; oder
$R^{13}$ und $R^{14}$ zusammen mit dem Stickstoffatom, an das sie gebunden sind, zyklisches Imid der Formel

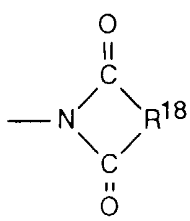

darstellen, dessen Ringstruktur 4 bis 6 Kohlenstoffatome enthält; und
$R^{18}$ $C_2$-$C_{18}$-Alkylen oder $C_2$-$C_6$-Alkenylen darstellt.

**3.** Verbindung der Formel I gemäß Anspruch 2, worin A Methylen ist, E die Bedeutung

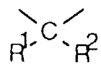

hat, und
$R^2$ -OH; -O-$R^8$; -OCO-$R^8$; -NHCO-$R^8$ oder -N($R^{13}$)$R^{14}$ ist; oder
$R^1$ und $R^2$ zusammen mit dem Kohlenstoffatom, an das sie gebunden sind, einen Fünf- oder Sechsring der Formel

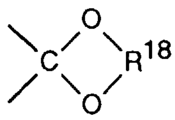 oder 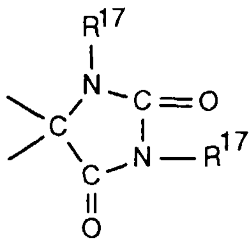

darstellen;
$R^4$, $R^5$, $R^6$ und $R^7$ Wasserstoff sind,
$R^8$ $C_1$-$C_{36}$-Alkyl, $C_2$-$C_8$-Alkenyl oder Glycidyl bedeutet; oder $C_2$-$C_{36}$-Alkyl bedeutet, welches durch -O-, -$NR^{19}$- und/oder $C_5$-$C_8$-Cycloalkylen unterbrochen ist; oder $R^8$ $C_5$-$C_{12}$-Cycloalkyl; Phenyl, welches unsubstituiert oder durch -$R^{12}$ oder -$OR^{12}$ substituiert ist; oder $C_7$-$C_9$-Aralkyl, welches unsubstituiert oder im aromatischen Teil durch -$R^{12}$ substituiert ist, bedeutet;
$R^{12}$ $C_1$-$C_8$-Alkyl; $C_2$-$C_8$-Alkenyl; Cyclohexyl; Phenyl oder Benzyl ist;
$R^{13}$ und $R^{14}$, unabhängig voneinander, jeweils eine der für $R^8$ angegebenen Bedeutungen haben; oder
$R^{13}$ und $R^{14}$ zusammen mit dem Stickstoffatom, an das sie gebunden sind, zyklisches Imid der Formel

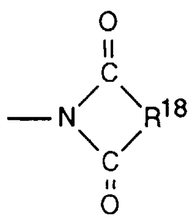

darstellen, dessen Ringstruktur 4 bis 6 Kohlenstoffatome enthält; und
$R^{18}$ $C_2$-$C_3$-Alkylen oder $C_2$-$C_3$-Alkenylen darstellt.

4. Verbindung der Formel I gemäß Anspruch 3, worin A Methvlen ist. E die Bedeutung

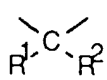

hat, und
$R^2$ -OH; -O-$R^8$; -OCO-$R^8$; -NHCO-$R^8$ oder -N($R^{13}$)$R^{14}$ ist; oder
$R^1$ und $R^2$ zusammen mit dem Kohlenstoffatom, an das sie gebunden sind, einen Fünf- oder Sechsring der Formel

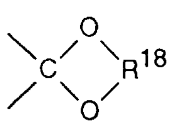 oder 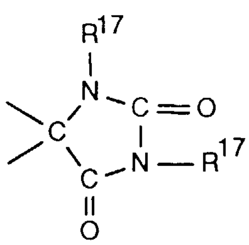

darstellen;
$R^4$, $R^5$, $R^6$ und $R^7$ Wasserstoff sind,
$R^8$ $C_1$-$C_{18}$-Alkyl, $C_2$-$C_8$-Alkenyl oder Glycidyl bedeutet; oder $R^8$ $C_5$-$C_{12}$-Cycloalkyl; Phenyl, welches unsubstituiert oder durch -$R^{12}$ substituiert ist; oder $C_7$-$C_9$-Aralkyl bedeutet;

$R^{12}$ $C_1$-$C_8$-Alkyl; Vinyl; α-Methylvinyl oder Allyl ist;
$R^{13}$ und $R^{14}$, unabhängig voneinander, jeweils eine der für $R^8$ angegebenen Bedeutungen haben.

**5.** Verbindung der Formel I gemäß Anspruch 4, worin n 1 oder 2 ist, A Methylen ist, E die Bedeutung

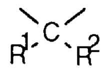

hat, und
$R^2$ -OH; $C_1$-$C_{18}$-Alkoxy; Allyloxy; $C_7$-$C_9$-Aralkoxy; Glycidyloxy; $C_2$-$C_{18}$-Alkanoyloxy; $C_3$-$C_4$-Alkenoyloxy; Benzoyloxy; Formamidyl; $C_2$-$C_{18}$-Alkanoylamido; Benzoylamido; oder -N($R^{13}$)$R^{14}$ ist; oder
$R^1$ und $R^2$ zusammen mit dem Kohlenstoffatom, an das sie gebunden sind, einen Fünf- oder Sechsring der Formel

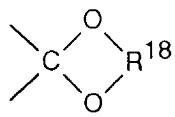 oder 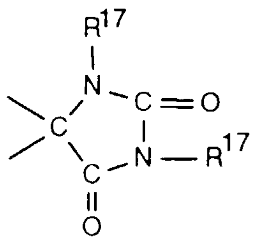

darstellen;
$R^4$, $R^5$, $R^6$ und $R^7$ Wasserstoff sind;
$R^{13}$ und $R^{14}$, unabhängig voneinander, $C_1$-$C_{18}$-Alkyl bedeuten; und $R_{18}$ $C_2$-$C_3$-Alkylen oder $C_2$-Alkenylen darstellt.

**6.** Verbindung der Formel I gemäß Anspruch 5, worin A Methylen ist, E die Bedeutung

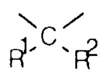

hat, und
$R^2$ -OH; $C_6$-$C_{18}$-Alkoxy; Allyloxy; Benzyloxy; Glycidyloxy; $C_2$-$C_{18}$-Alkanoyloxy; oder $C_3$-$C_4$-Alkenoyloxy ist; oder
$R^1$ und $R^2$ zusammen mit dem Kohlenstoffatom, an das sie gebunden sind, einen Fünfring der Formel

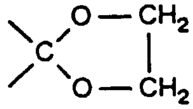 oder 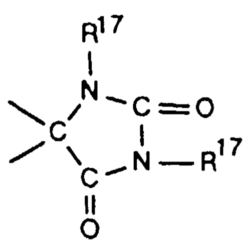

darstellen; und
$R^4$, $R^5$, $R^6$ und $R^7$ Wasserstoff sind.

**7.** Verbindung der Formel

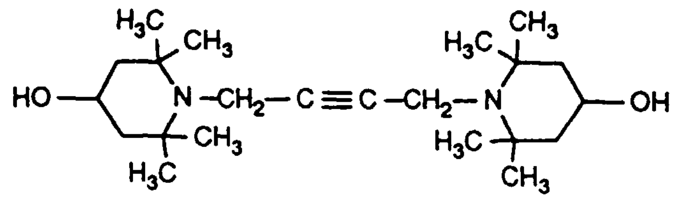

gemäß Anspruch 6.

**8.** Zusammensetzung enthaltend (a) ein gegen oxidativen, thermischen oder/und aktinischen Abbau empfindliches organisches Material und (b) mindestens eine Verbindung der Formel I gemäß Anspruch 1.

**9.** Zusammensetzung gemäß Anspruch 8 enthaltend als Komponente (a) ein synthetisches organisches Polymer.

**10.** Zusammensetzung gemäß Anspruch 8 enthaltend 0,01 bis 10 % der Verbindung der Komponente (b), bezogen auf das Gesamtgewicht der stabilisierten Zusammensetzung.

**11.** Zusammensetzung gemäß Anspruch 8 enthaltend als zusätzliche Komponente (c) ein oder mehrere herkömmliche Additive.

**12.** Verwendung von Verbindungen der Formel I gemäß Anspruch 1 zum Stabilisieren von organischem Material gegen oxidativen, thermischen oder aktinischen Abbau.

**13.** Verfahren zum Stabilisieren von organischem Material gegen thermischen, oxidativen oder/und aktinischen Abbau, dadurch gekennzeichnet, daß man diesem Material mindestens eine Verbindung der Formel I gemäß Anspruch 1 zusetzt.

**14.** Verfahren zur Herstellung einer Verbindung der Formel I gemäß Anspruch 1, dadurch gekennzeichnet, daß man einen Ester einer aromatischen Sulfonsäure der Formel II

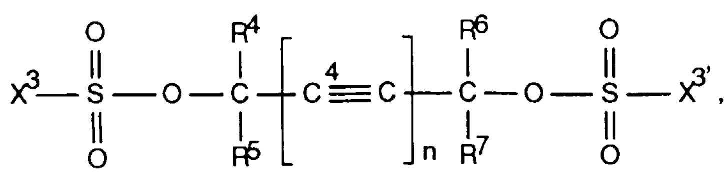 (II)

worin $X^3$ und $X^{3'}$, unabhängig voneinander, Phenyl oder durch $C_1$-$C_8$-Alkyl, $C_1$-$C_8$-Alkoxy oder Halogen substituiertes Phenyl sind,
mit einer Verbindung der Formel III

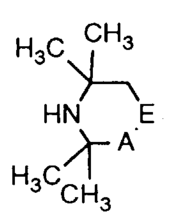 (III)

umsetzt.

**15.** Verfahren gemäß Anspruch 14, worin die Umsetzung in einem polaren organischen Lösungsmittel durchgeführt wird.

## Claims

**1.** A compound of the formula I

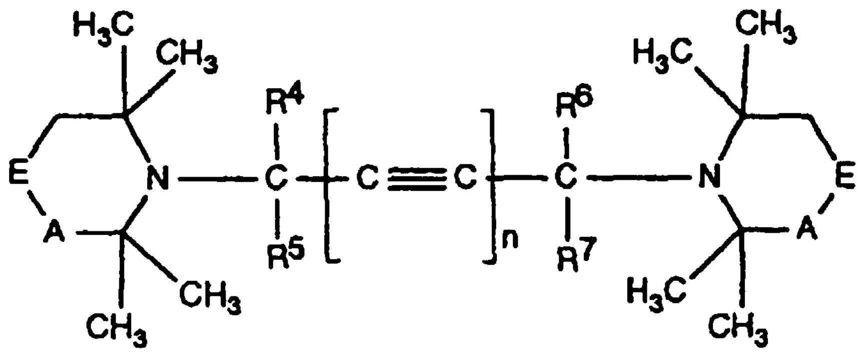 (I)

wherein n is 1 or 2, A is $-CH_2-$ or -CO-;

if A is methylene, E is

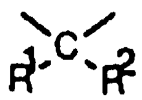,

and

if A is carbonyl, E is $>N-R^3$;

$R^1$ is hydrogen;

$R^2$ is -OH; $-O-R^8$; $-S-R^8$; $-OCO-R^8$; $-NHCO-R^8$ or $-N(R^{13})R^{14}$; or

$R^1$ and $R^2$, taken together, are a substituent =0 or, together with the carbon atom to which they are attached, are a five- or six-membered ring of one of the formulae

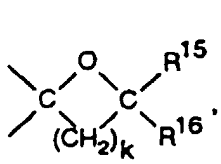, 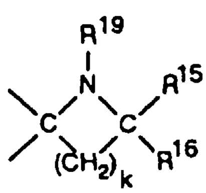, 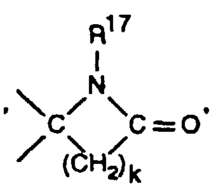, 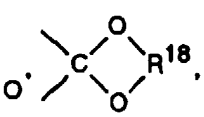,

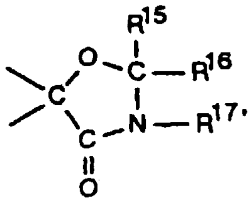, 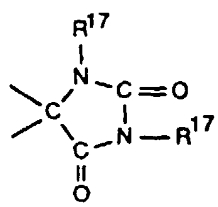,

where k is 2 or 3;

$R^3$ is $C_1$-$C_{18}$alkyl; $C_3$-$C_{18}$alkenyl; $C_5$-$C_8$cycloalkyl; phenyl; naphthyl; $C_7$-$C_9$phenylalkyl; or $C_{11}$-$C_{14}$naphthylalkyl;

$R^4$, $R^5$, $R^6$ and $R^7$ are each independently of one another hydrogen or methyl;

$R^8$ is $C_1$-$C_{50}$alkyl, $C_2$-$C_{18}$alkenyl or glycidyl; or $C_2$-$C_{50}$alkyl which is interrupted by -O-, -S-, -$NR^{19}$-and/or $C_5$-$C_8$cycloalkylene; or $R^8$ is $C_5$-$C_{12}$cycloalkyl which is unsubstituted or substituted by 1 to 4 substituents -$R^{12}$; $C_6$-$C_{10}$aryl which is unsubstituted or substituted by 1 to 4 substituents -$OR^{12}$; or $C_7$-$C_{50}$aralkyl which is unsubstituted or substituted by $C_5$-$C_8$cycloalkyl and/or interrupted in the aliphatic moiety by $C_5$-$C_8$cycloalkylene and/or by oxygen or sulfur, and/or substituted in the aromatic moiety by 1 to 4 substituents -$R^{12}$ or -$OR^{12}$;

$R^{12}$ is $C_1$-$C_{18}$alkyl; $C_2$-$C_{18}$alkenyl; $C_5$-$C_8$cycloalkyl; phenyl or benzyl;

$R^{13}$ and $R^{14}$, each independently of the other, have one of the meanings given for $R^8$; or

$R^{13}$ and $R^{14}$, together with the nitrogen atom to which they are attached, are cyclic imide of the formula

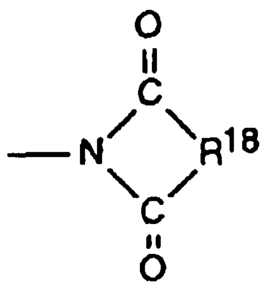

whose ring structure contains 4 to 6 carbon atoms;

$R^{15}$ and $R^{16}$, each independently of the other, are H; or $C_1$-$C_{12}$alkyl; or, taken together, are straight-chain $\alpha$, $\omega$-linked $C_4$-$C_{13}$alkylene;

$R^{17}$ is H or has one of the meanings of $R^3$;

$R^{18}$ is $C_2$-$C_{18}$alkylene or $C_2$-$C_6$alkenylene; and

$R^{19}$ has one of the meanings of $R^3$.

**2.** A compound of the formula I according to claim 1, wherein

A is methylene, E is

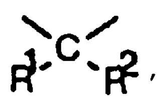

and

$R^2$ is -OH; -O-$R^8$; -OCO-$R^8$; -NHCO-$R^8$ or -N($R^{13}$)$R^{14}$; or

$R^1$ and $R^2$, taken together, are a substituent =O or, together with the carbon atom to which they are attached, are a five- or six-membered ring of one of the formulae

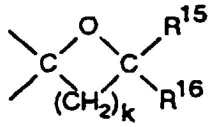, 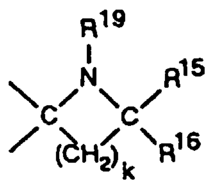, 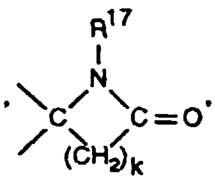, 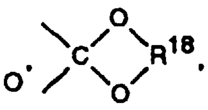,

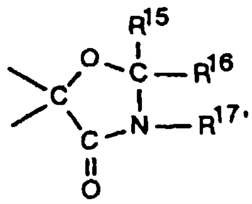, 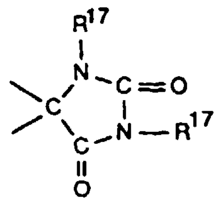,

where k is 2 or 3;

$R^4$, $R^5$, $R^6$ and $R^7$ are hydrogen;

$R^8$ is $C_1$-$C_{50}$alkyl, $C_2$-$C_{18}$alkenyl or glycidyl; or $C_2$-$C_{50}$alkyl which is interrupted by -O-, -S-, -$NR^{19}$-and/or $C_5$-$C_8$cycloalkylene; or $R^8$ is $C_5$-$C_{12}$cycloalkyl which is unsubstituted or substituted by 1 to 4 substituents -$R^{12}$; phenyl which is unsubstituted or substituted by 1 to 4 substituents -$R^{12}$ or -$OR^{12}$; or $C_7$-$C_{18}$aralkyl which is unsubstituted or substituted in the aromatic moiety by 1 to 4 substituents -$R^{12}$ or -$OR^{12}$;

$R^{12}$ is $C_1$-$C_{18}$alkyl; $C_2$-$C_{18}$alkenyl; $C_5$-$C_8$cycloalkyl; phenyl or benzyl;

$R^{13}$ and $R^{14}$, each independently of the other, have one of the meanings given for $R^8$; or

$R^{13}$ and $R^{14}$, together with the nitrogen atom to which they are attached, are cyclic imide of the formula

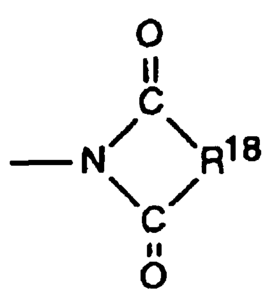

whose ring structure contains 4 to 6 carbon atoms; and $R^{18}$ is $C_2$-$C_{18}$alkylene or $C_2$-$C_6$alkenylene.

**3.** A compound of the formula I according to claim 2, wherein A is methylene, E is

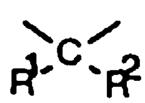,

and

$R^2$ is -OH; -O-$R^8$; -OCO-$R^8$; -NHCO-$R^8$ or -N($R^{13}$)$R^{14}$; or

$R^1$ and $R^2$, together with the carbon atom to which they are attached, are a five- or six-membered ring of the formula

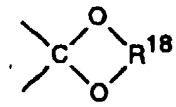 or 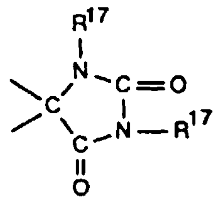 ;

$R^4$, $R^5$, $R^6$ and $R^7$ are hydrogen;

$R^8$ is $C_1$-$C_{36}$alkyl, $C_2$-$C_8$alkenyl or glycidyl; or $C_2$-$C_{36}$alkyl which is interrupted by -O-, -$NR^{19}$- and/or $C_5$-$C_8$cycloalkylene; or $R^8$ is $C_5$-$C_{12}$cycloalkyl; phenyl which is unsubstituted or substituted by -$R^{12}$ or -$OR^{12}$;

or $C_7$-$C_9$aralkyl which is unsubstituted or substituted in the aromatic moiety by -$R^{12}$;

$R^{12}$ is $C_1$-$C_8$alkyl; $C_2$-$C_8$alkenyl; cyclohexyl; phenyl or benzyl;

$R^{13}$ and $R^{14}$, each independently of the other, have one of the meanings given for $R^8$; or

$R^{13}$ and $R^{14}$, together with the nitrogen atom to which they are attached, are cyclic imide of the formula

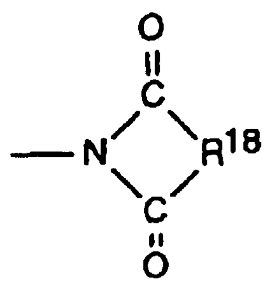

whose ring structure contains 4 to 6 carbon atoms; and $R^{18}$ is $C_2$-$C_3$alkylene or $C_2$-$C_3$alkenylene.

**4.** A compound of the formula I according to claim 3, wherein

A is methylene, E is

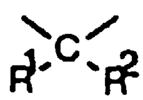 ,

and
$R^2$ is -OH; -O-$R^8$; -OCO-$R^8$; -NHCO-$R^8$ or -N($R^{13}$)$R^{14}$; or
$R^1$ and $R^2$, together with the carbon atom to which they are attached, are a five- or six-membered ring of the formula

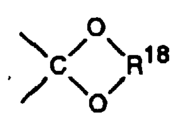 or 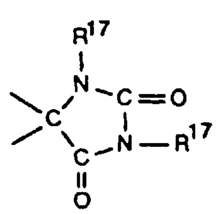 ;

$R^4$, $R^5$, $R^6$ and $R^7$ are hydrogen;
$R^8$ is $C_1$-$C_{18}$alkyl, $C_2$-$C_8$alkenyl or glycidyl; or $R^8$ is $C_5$-$C_{12}$cycloalkyl; phenyl which is unsubstituted or substituted by -$R^{12}$; or $C_7$-$C_9$aralkyl;
$R^{12}$ is $C_1$-$C_8$alkyl; vinyl; $\alpha$-methylvinyl or allyl;
$R^{13}$ and $R^{14}$, each independently of the other, have one of the meanings given for $R^8$.

**5.** A compound of the formula I according to claim 4, wherein n is 1 or 2, A is methylene, E is

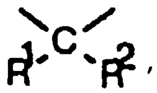

and

$R^2$ is -OH; $C_1$-$C_{18}$alkoxy; allyloxy; $C_7$-$C_9$aralkoxy; glycidyloxy; $C_2$-$C_{18}$alkanoyloxy; $C_3$-$C_4$alkenoyloxy; benzoyloxy; formamidyl; $C_2$-$C_{18}$alkanoylamido; benzoylamido; or -N($R^{13}$)$R^{14}$; or
$R^1$ and $R^2$, together with the carbon atom to which they are attached, are a five- or six-membered ring of the formula

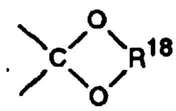 or 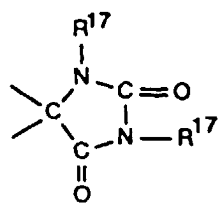 ;

$R^4$, $R^5$, $R^6$ and $R^7$ are hydrogen;
$R^{13}$ and $R^{14}$, each independently of the other, are $C_1$-$C_{18}$alkyl; and $R^{18}$ is $C_2$-$C_3$alkylene or $C_2$alkenylene.

**6.** A compound of the formula I according to claim 5, wherein A is methylene, E is

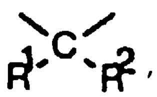

and

$R^2$ is -OH; $C_6$-$C_{18}$alkoxy; allyloxy; benzyloxy; glycidyloxy; $C_2$-$C_{18}$alkanoyloxy; or $C_3$-$C_4$alkenoyloxy; or $R^1$ and $R^2$, together with the carbon atom to which they are attached, are a five-membered ring of the formula

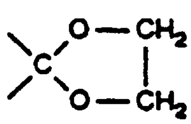 or 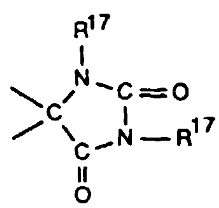 ;

and

$R^4$, $R^5$, $R^6$ and $R^7$ are hydrogen.

**7.** A compound of the formula

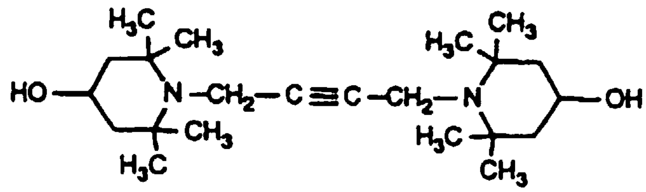

according to claim 6.

**8.** A composition comprising (a) an organic material susceptible to oxidative, thermal and/or actinic degradation, and (b) at least one compound of the formula I according to claim 1.

**9.** A composition according to claim 8, comprising as component (a) a synthetic organic polymer.

**10.** A composition according to claim 8, comprising 0.01 to 10% of the compound of component (b), based on the total weight of the stabilised composition.

**11.** A composition according to claim 8, comprising as additional component (c) one or more than one conventional additive.

**12.** The use of a compound of the formula I according to claim 1 for stabilising organic material against oxidative, thermal or actinic degradation.

**13.** A process for stabilising organic material against thermal, oxidative and/or actinic degradation, which comprises adding at least one compound of the formula I according to claim 1 to this material.

**14.** A process for preparing a compound of the formula I according to claim 1, which comprises reacting an ester of an aromatic sulfonic acid of the formula II

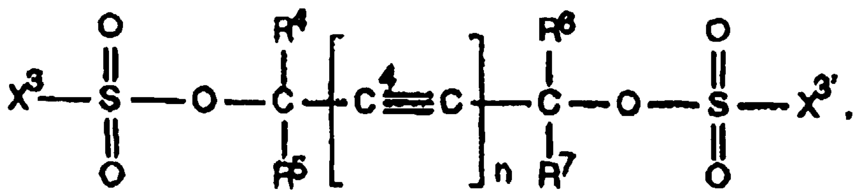

(II) ,

wherein $X^3$ and $X^{3'}$, each independently of the other, are phenyl or phenyl which is substituted by $C_1$-$C_8$alkyl, $C_1$-$C_8$alkoxy or halogen,
with a compound of the formula III

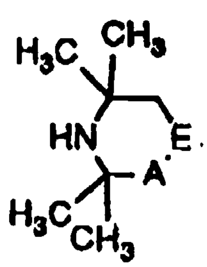

(III) .

**15.** A process according to claim 14, wherein the reaction is carried out in a polar organic solvent.

**Revendications**

**1.** Composé de formule I

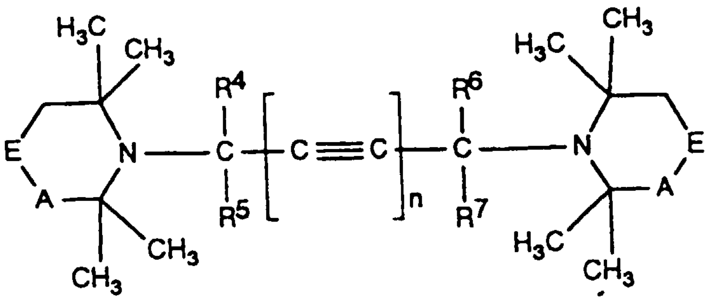

(I)

où

n vaut 1 ou 2,

A représente des groupes $-CH_2-$ ou -CO- ;

lorsque A représente un groupe méthylène,
E représente

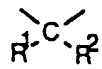

,

et
lorsque A représente un groupe carbonyle, E représente $>N-R^3$ ;

$R^1$ représente un atome d'hydrogène ;

$R^2$ représente des groupes -OH ; $-O-R^8$ ; $-S-R^8$ ; $-OCO-R^8$, $-NHCO-R^8$ ou $-N(R^{13})R^{14}$ ; ou

$R^1$ et $R^2$ représentent ensemble un substituant =O ou conjointement avec l'atome de carbone auquel ils sont liés, représentent un cycle à 5 ou 6 chaînons d'une des formules

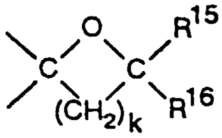

,

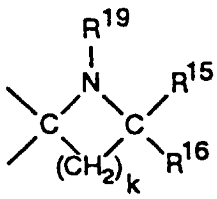

,

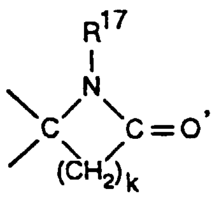

,

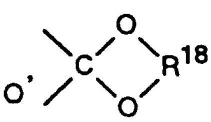

,

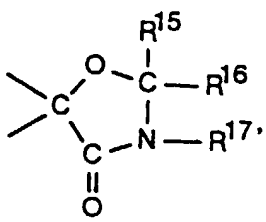

,

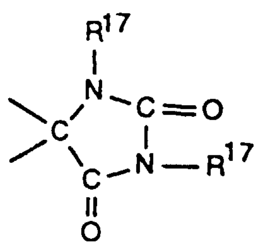

,

où k vaut 2 ou 3 ;

$R^3$ représente des groupes alkyle en $C_1-C_{18}$ ; alcényle en $C_3-C_{18}$ ; cycloalkyle en $C_5-C_8$, phényle ; naphtyle ; phénylalkyle en $C_7-C_9$ ; ou naphtylalkyle en $C_{11}-C_{14}$ ;

$R^4$, $R^5$, $R^6$ et $R^7$ représentent, indépendamment les uns des autres, des atomes d'hydrogène ou des grou-

pes méthyle ;

$R^8$ représente des groupes alkyle en $C_1$-$C_{50}$, alcényle en $C_2$-$C_{18}$ ou glycidyle ; ou alkyle en $C_2$-$C_{50}$ interrompu par des groupes -O-, -S-, -$NR^{19}$- et/ou cycloalkylène en $C_5$-$C_8$ ; ou $R^8$ représente un groupe cycloalkyle en $C_5$-$C_{12}$ non substitué ou substitué par 1 à 4 substituants -$R^{12}$ ; aryle en $C_6$-$C_{10}$ non substitué ou substitué par 1 à 4 substituants -$R^{12}$ ou -$OR^{12}$ ; ou aralkyle en $C_7$-$C_{50}$ non substitué ou substitué par un groupe cycloalkyle en $C_5$-$C_8$ et/ou interrompu dans le fragment aliphatique par un groupe cycloalkylène en $C_5$-$C_8$ et/ou interrompu dans le fragment aliphatique par des atomes d'oxygène ou de soufre et/ou substitué dans le fragment aromatique par 1 à 4 substituants -$R^{12}$ ou -$OR^{12}$ ;

$R^{12}$ représente des groupes alkyle en $C_1$-$C_{18}$ ; alcényle en $C_2$-$C_{18}$ ; cycloalkyle en $C_5$-$C_8$ ; phényle oui benzyle ;

$R^{13}$ et $R^{14}$ possèdent chacun, indépendamment l'un de l'autre, une des significations données pour $R^8$ ; ou

$R^{13}$ et $R^{14}$ représentent, ensemble avec l'atome d'azote auquel ils sont liés, un imide cyclique de formule

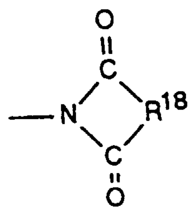

dont la structure cyclique présente 4 à 6 atomes de carbone ;

$R^{15}$ et $R^{16}$ représentent, indépendamment l'un de l'autre, des atomes d'hydrogène ; ou des groupes alkyle en $C_1$-$C_{12}$ ; ou ensemble, un groupe alkylène en $C_4$-$C_{13}$ à chaîne droite, relié en $\alpha,\omega$ ;

$R^{17}$ représente un atome d'hydrogène ou possède une des significations de $R^3$; et

$R^{18}$ représente des groupes alkylène en $C_2$-$C_{18}$ ou alcénylène en $C_2$-$C_6$ ; et

$R^{19}$ possède une des significations données pour $R^3$.

**2.** Composé de formule I selon la revendication 1, où

A représente un groupe méthylène,

E représente un groupe

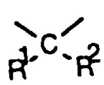 ,

et

$R^2$ représente des groupes -OH ; -O-$R^8$ ; -OCO-$R^8$, -NHCO-$R^8$ ou -N($R^{13}$)$R^{14}$ ; ou

$R^1$ et $R^2$ représentent ensemble un substituant =O ou conjointement avec l'atome de carbone auquel ils sont liés, représentent un cycle à 5 ou 6 chaînons d'une des formules

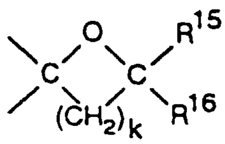 , 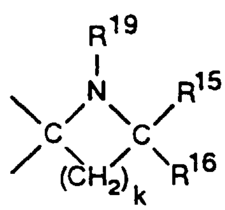 , 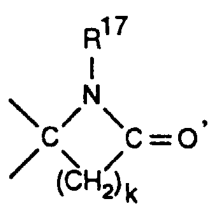 , 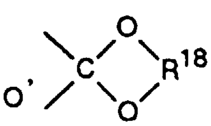 ,

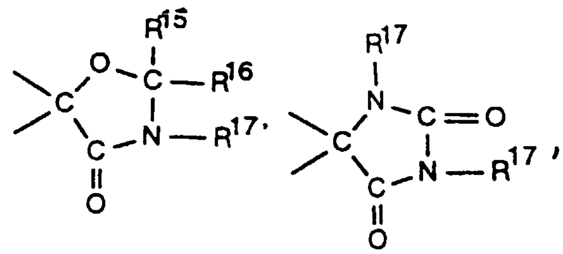

où k vaut 2 ou 3 ;

$R^4$, $R^5$, $R^6$ ou $R^7$ représentent des atomes d'hydrogène,

$R^8$ représente des groupes alkyle en $C_1$-$C_{50}$, alcényle en $C_2$-$C_{18}$ ou glycidyle ; ou alkyle en $C_2$-$C_{50}$ interrompu par des groupes -O-, -S-, -$NR^{19}$- et/ou cycloalkylène en $C_5$-$C_8$ ; ou $R^8$ représente un groupe cycloalkyle en $C_5$-$C_{12}$ non substitué ou substitué par 1 à 4 substituants -$R^{12}$ ; phényle non substitué ou substitué par 1 à 4 substituants -$R^{12}$ ou -$OR^{12}$ ; ou aralkyle en $C_7$-$C_{18}$ non substitué ou substitué dans le fragment aromatique par 1 à 4 substituants -$R^{12}$ ou -$OR^{12}$ ;

$R^{12}$ représente des groupes alkyle en $C_1$-$C_{18}$ ; alcényle en $C_2$-$C_{18}$ ; cycloalkyle en $C_5$-$C_8$, phényle ou benzyle ;

$R^{13}$ et $R^{14}$ possèdent chacun, indépendamment l'un de l'autre, une des significations données pour $R^8$ ; ou

$R^{13}$ et $R^{14}$ représentent, conjointement avec l'atome d'azote auquel ils sont liés, un imide cyclique de formule

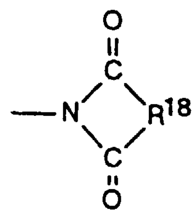

dont la structure cyclique présente 4 à 6 atomes de carbone ;

$R^{18}$ représente des groupes alkylène en $C_2$-$C_{18}$ ou alcénylène en $C_2$-$C_6$.

**3.** Composé de formule I selon la revendication 2, où

A représente un groupe méthylène,

E possède la signification

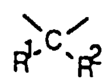

et

$R^2$ représente des groupes -OH ; -O-$R^8$ ; -OCO-$R^8$, -NHCO-$R^8$ ou -N($R^{13}$)$R^{14}$ ; ou

$R^1$ et $R^2$ forment conjointement avec l'atome de carbone auquel ils sont liés, un cycle à 5 ou 6 chaînons de formule

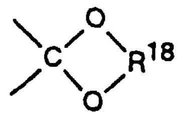 ou 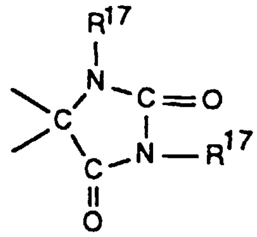 ;

$R^4$, $R^5$, $R^6$ ou $R^7$ représentent des atomes d'hydrogène,

$R^8$ représente des groupes alkyle en $C_1$-$C_{36}$, alcényle en $C_2$-$C_8$ ou glycidyle ; ou alkyle en $C_2$-$C_{36}$ interrompu par des groupes -O-, -$NR^{19}$- et/ou cycloalkylène en $C_5$-$C_8$ ; ou $R^8$ représente un groupe cycloalkyle en $C_5$-$C_{12}$ ; phényle non substitué ou substitué par des substituants -$R^{12}$ ou -$OR^{12}$ ; ou aralkyle en $C_7$-$C_9$ non substitué ou substitué dans le fragment aromatique par un substituant -$R^{12}$ ;

$R^{12}$ représente des groupes alkyle en $C_1$-$C_{18}$ ; alcényle en $C_2$-$C_{18}$ ; cyclohexyle ; phényle ou benzyle ;

$R^{13}$ et $R^{14}$ possèdent chacun, indépendamment l'un de l'autre, une des significations données pour $R^8$ ; ou

$R^{13}$ et $R^{14}$ représentent, conjointement avec l'atome d'azote auquel ils sont liés, un imide cyclique de formule

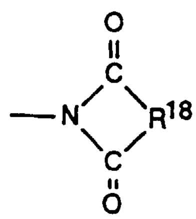

dont la structure cyclique contient 4 à 6 atomes de carbone ;

$R^{18}$ représente des groupes alkylène en $C_2$-$C_3$ ou alcénylène en $C_2$-$C_3$.

**4.** Composé de formule I selon la revendication 3, où

A représente un groupe méthylène,

E possède la signification

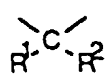 ,

et

$R^2$ représente des groupes -OH ; -O-$R^8$ ; -OCO-$R^8$, -NHCO-$R^8$ ou -N($R^{13}$)$R^{14}$ ; ou

$R^1$ et $R^2$ forment conjointement avec l'atome de carbone auquel ils sont liés, un cycle à 5 ou 6 chaînons de formule

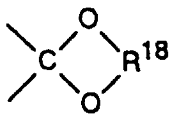 ou 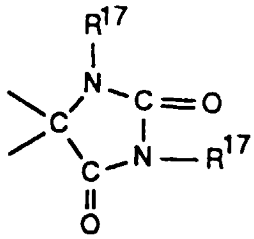 ;

| | |
|---|---|
| $R^4$, $R^5$, $R^6$ ou $R^7$ | représentent des atomes d'hydrogène, |
| $R^8$ | représente des groupes alkyle en $C_1$-$C_{18}$, alcényle en $C_2$-$C_8$ ou glycidyle ; ou $R^8$ représente un groupe cycloalkyle en $C_5$-$C_{12}$ ; phényle non substitué ou substitué par un substituant -$R^{12}$ ; ou aralkyle en $C_7$-$C_9$ ; |
| $R^{12}$ | représente des groupes alkyle en $C_1$-$C_8$ ; vinyle ; $\alpha$-méthylvinyle ou allyle ; |
| $R^{13}$ et $R^{14}$ | possèdent chacun, indépendamment l'un de l'autre, une des significations données pour $R^8$. |

**5.** Composés de formule I selon la revendication 4, où

| | |
|---|---|
| A | représente un groupe méthylène, |
| E | possède la signification |

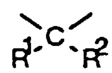 ,

et

| | |
|---|---|
| $R^2$ | représente des groupes -OH ; alkoxy en $C_1$-$C_{18}$ ; allyloxy ; aralkoxy en $C_7$-$C_9$ ; glycidyloxy ; alcanoyloxy en $C_2$-$C_{18}$ ; alcénoyloxy en $C_3$-$C_4$ ; benzoyloxy ; formamidyle ; (alcanoyl en $C_2$-$C_{18}$)-amido ; benzoylamido ou -N($R^{13}$)$R^{14}$ ; ou |
| $R^1$ et $R^2$ | forment conjointement avec l'atome de carbone auquel ils sont liés, un cycle à 5 ou 6 chaînons de formule |

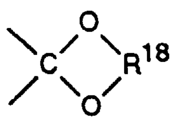 ou 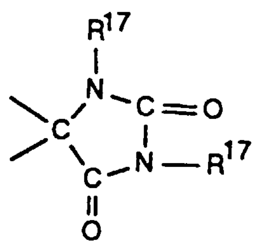 ;

| | |
|---|---|
| $R^4$, $R^5$, $R^6$ ou $R^7$ | représentent des atomes d'hydrogène ; |
| $R^{13}$ et $R^{14}$ | possèdent chacun, indépendamment l'un de l'autre, des groupes alkyle en $C_1$-$C_{18}$ ; et $R^{18}$ représente des groupes alkylène en $C_2$-$C_3$ ou alcénylène en $C_2$. |

**6.** Composé de formule I selon la revendication 5 où

| | |
|---|---|
| A | représente un groupe méthylène, |
| E | possède la signification |

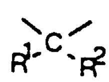 ,

et

| | |
|---|---|
| $R^2$ | représente des groupes -OH ; alkoxy en $C_6$-$C_{18}$ ; allyloxy ; benzyloxy ; glycidyloxy ; alca- |

noyloxy en $C_2$-$C_{18}$ ; ou alcénoyloxy en $C_3$-$C_4$ ; ou

$R^1$ et $R^2$ forment, conjointement avec l'atome de carbone auquel ils sont liés, un cycle à 5 ou 6 chaînons de formule

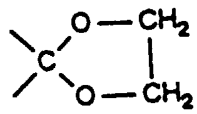

ou

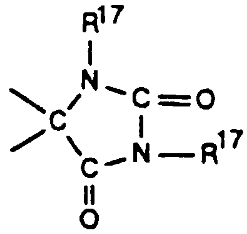

;

$R^4$, $R^5$, $R^6$ ou $R^7$ représentent des atomes d'hydrogène.

**7.** Composé de formule

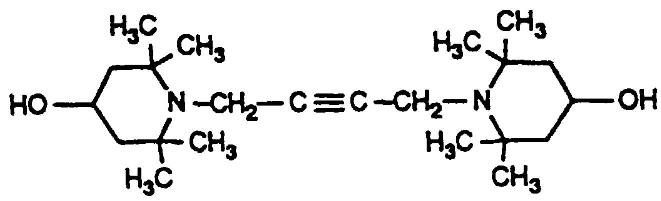

selon la revendication 6.

**8.** Composition contenant (a) une matière sensible à la dégradation oxydative, thermique ou/et actinique et (b) au moins un composé de formule I selon la revendication 1.

**9.** Composition selon la revendication 8, contenant en tant que constituant (a) un polymère organique synthétique.

**10.** Compositions selon la revendication 8, contenant de 0,01 à 10 % du composé du constituant (b), par rapport au poids total de la composition à stabiliser.

**11.** Composition selon la revendication 8, contenant en tant que constituant supplémentaire (c) un ou plusieurs additifs classiques.

**12.** Utilisation de composés de formule I selon la revendication 1, pour la stabilisation de matière organique contre la dégradation oxydative, thermique ou/et actinique.

**13.** Procédé pour la stabilisation de matière organique contre la dégradation thermique, oxydative ou/et actinique, caractérisée en ce qu'on ajoute à cette matière au moins un composé de formule I selon la revendication 1.

**14.** Procédé pour la préparation d'un composé de formule I selon la revendication 1, caractérisé en ce qu'on fait réagir un ester d'un acide sulfonique de formule II

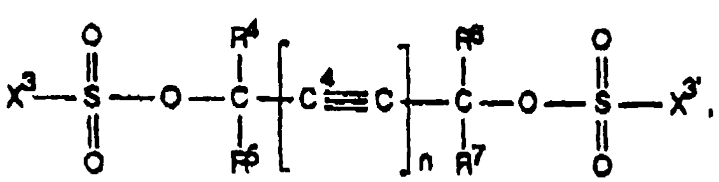

(II)

où $X^3$ et $X^{3'}$ représentent, indépendamment l'un de l'autre, des groupes phényle ou phényle substitué par des substituants alkyle en $C_1$-$C_8$, alkoxy en $C_1$-$C_8$ ou halogène,

sur un composé de formule III

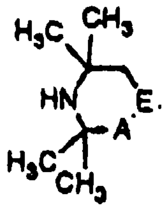

(III).

**15.** Procédé selon la revendication 14, où la réaction est mise en oeuvre dans un solvant organiques polaire.